(19)

Europäisches
Patentamt
European
Patent Office
Office européen
des brevets

(11)    **EP 4 495 250 A1**

(12)    **EUROPEAN PATENT APPLICATION**

(43) Date of publication:
22.01.2025   Bulletin 2025/04

(21) Application number: 23186083.4

(22) Date of filing: 18.07.2023

(51) International Patent Classification (IPC):
*C12Q 1/6806* (2018.01)   *C12N 15/10* (2006.01)

(52) Cooperative Patent Classification (CPC):
(C-Sets available)
C12Q 1/6806; C12N 15/1003               (Cont.)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(71) Applicant: Centogene GmbH
18055 Rostock (DE)

(72) Inventors:
• Bauer, Peter
18211 Börgerende-Rethwisch (DE)

• Beetz, Christian
18107 Elmenhorst/Lichtenhagen (DE)
• Al-Ali, Ruslan
18146 Rostock (DE)
• Radefeldt, Mandy
18055 Rostock (DE)
• Lemke, Sabrina
18146 Rostock (DE)

(74) Representative: Bohmann, Armin K.
Bohmann
Anwaltssozietät
Nymphenburger Straße 1
80335 München (DE)

(54)    **METHOD FOR PREPARING AN RNA PREPARATION AND USE THEREOF**

(57)    The present invention is related to a method for preparing an RNA preparation, wherein the method comprises
- extracting an RNA from a blood optionally comprising blood cells contained in a first sample and/or extracting an RNA from blood cells contained in a first sample, wherein the RNA extracted from the blood optionally comprising blood cells and/or the RNA extracted from the blood cells is provided as a second sample, and
- depleting an RNA from the second sample, wherein the RNA obtained after depleting the RNA from the second sample, is provided as the RNA preparation.

**EP 4 495 250 A1**

(52) Cooperative Patent Classification (CPC): (Cont.)

C-Sets
**C12Q 1/6806, C12Q 2523/10;**
**C12Q 1/6806, C12Q 2523/10, C12Q 2523/303**

**Description**

The present invention is related to a method for preparing an RNA preparation, a method of sequencing an RNA molecule in a sample using such RNA preparation, a method for quantifying an RNA molecule in a sample using such RNA preparation, a method for determining the course of a disease in a subject using such RNA preparation, a method for monitoring an effect of a therapy administered to a subject suffering from a disease using such RNA preparation, a method for determining whether a subject suffering from a disease is likely to respond to a treatment using such RNA preparation, a method for determining the presence or absence of a splice variant of a gene in a sample using such RNA preparation, a method for determining a relative abundance of an individual RNA molecule in a sample using such RNA preparation, a method for determining allele-specific expression of a gene in a plurality of samples, and a method for determining the proportion of subjects of a group of subjects carrying an allele of a gene using such RNA preparation.

The transcription of a subset of genes into complementary RNA molecules specifies a cell's identity and regulates the biological activities within the cell. Collectively defined as the transcriptome, these RNA molecules are essential for interpreting the functional elements of the genome and understanding development and disease.

In accordance with the central dogma of molecular biology, the transcriptome initially encompassed only protein-coding mRNA transcripts. Nevertheless, several RNA subtypes with distinct functions exist. Many RNA transcripts do not code for protein or have different regulatory functions in the process of gene transcription and translation. RNA types which do not fall within the scope of the central dogma of molecular biology are non-coding RNAs which can be divided into two groups of long non-coding RNA and short non-coding RNA.

Long non-coding RNA includes all non-coding RNA transcripts that are more than 200 nucleotides long. Members of this group comprise the largest fraction of the non-coding transcriptome. Short non-coding RNA includes the following members: transfer RNA (tRNA), micro RNA (miRNA), Micro RNAs, small interfering RNA (siRNA), small nucleolar RNA (snoRNA), Piwi-interacting RNA (piRNA) and enhancer RNA (eRNA).

The development of high-throughput next-generation sequencing (also called massively parallel sequencing and NGS which is a high-throughput method used to determine a portion of the nucleotide sequence of an individual's genome) has revolutionized transcriptomics by enabling RNA analysis through the sequencing of complementary DNA (cDNA). This method, termed RNA sequencing (abbr. RNA-Seq), has eliminated many challenges posed by hybridization-based microarrays and Sanger sequencing-based approaches that were previously used for measuring gene expression and has revolutionized the understanding of the complex and dynamic nature of the transcriptome. RNA-Seq provides a more detailed and quantitative view of gene expression, alternative splicing, and allele-specific expression. Advances in the RNA-Seq workflow, from sample preparation to sequencing platforms to bioinformatic data analysis, has enabled deep profiling of the transcriptome and the opportunity to elucidate different physiological and pathological conditions. Greater quantities of high-quality RNA (RNA Integrity Number [RIN] > 7) are ideal.

Over the past decades, dried blood spot (abbr. DBS) technology has become a convenient tool in both qualitative and quantitative laboratory analysis. Its applicability saw a significant expansion in recent decades, with a shift from basic to clinical research and medicine. Compared with venipuncture, DBS offers a cost-effective solution, simplifying logistics for collecting, preserving, and transporting blood specimens in settings with minimal infrastructure.

Although DBS are widely used for diagnosis, in particular for the diagnosis for hereditary diseases, such use of DBS in diagnostics is mainly limited to DNA sequencing (genetic testing), biomarkers (if the biomarker is to be analyzed is stable) and enzymatic analysis (if the enzyme to be analyzed is stable). Only few working samples show the use of RNA-seq from DBS for research analysis, due to the low stability of the RNA and the limited amount of RNA, in particular mRNA, obtained from DBS, which can affect the accuracy and sensitivity of the RNA-seq analysis.

In light thereof, the problem underlying the present invention is the provision of a method for preparing an RNA preparation that leads to sufficient amounts of RNA, in particular mRNA, with sufficient quality that allows its use for RNA-Seq, gene expression analysis and diagnosis of diseases, in particular rare diseases.

A further problem underlying the present invention is the provision of an RNA preparation which is suitable for various applications and further processing steps, whereby such RNA preparation is obtained from a blood sample which can be easily stored and shows no relevant change in its composition, at least no relevant change which interferes with the RNA preparation's application and further processing steps to which the RNA preparation is subjected. Such application encompasses the use in the field of biotechnology, biochemistry, medicine, therapy and diagnosis.

These and other problems are solved by the subject matter of the attached independent claims; preferred embodiments may be taken from the attached dependent claims.

In addition, these and other problems are solved by the subject matter of the following embodiments.

Embodiment 1. A method for preparing an RNA preparation, wherein the method comprises

- extracting an RNA from a blood optionally comprising blood cells, wherein the blood is contained in a first sample, and/or extracting an RNA from blood cells contained in a first sample, wherein the RNA extracted from the blood optionally comprising blood cells and/or the RNA extracted from the blood cells is provided as a second sample, and

- depleting an RNA from the second sample, wherein the RNA obtained after depleting the RNA from the second sample, is provided as the RNA preparation,

wherein

the first sample is a dried blood sample comprising the blood optionally comprising blood cells and/or the first sample is a dried blood sample comprising the blood cells,

extracting the RNA from the blood optionally comprising blood cells and/or extracting the RNA from the blood cells comprises lysing the blood cells optionally comprised by the blood and/or lysing the blood cells, digesting protein contained in the first sample and/or digesting protein released from the blood cells optionally comprised by the blood and/or from the blood cells, and protecting from degradation RNA released from the blood cells optionally comprised by the blood and/or from the blood cells, and

depletion of the RNA from the second sample comprises depletion of hemoglobin RNA, depletion of rRNA and/or capturing of RNA by poly(A) tail capturing.

Embodiment 2. The method of Embodiment 1, wherein depleting the RNA from the second sample comprises depleting hemoglobin RNA, depleting rRNA and capturing of RNA by poly(A) tail capturing.

Embodiment 3. The method of any one of Embodiments 1 to 2, wherein the RNA preparation comprises the RNA captured by poly(A) tailing and wherein the RNA preparation is depleted from hemoglobin RNA and rRNA.

Embodiment 4. The method of any one of Embodiments 1 to 3, wherein the RNA preparation is a preparation comprising an mRNA, preferably the RNA preparation is an mRNA preparation.

Embodiment 5. The method of any one of Embodiments 1 to 4, wherein the hemoglobin RNA is depleted by binding the hemoglobin RNA contained in the second sample to an immobilized oligonucleotide that hybridize to hemoglobin RNA, preferably the immobilized oligonucleotide is an immobilized DNA oligonucleotide.

Embodiment 6. The method of any one of Embodiments 1 to 5, wherein the rRNA is depleted from the second sample by binding the rRNA to an immobilized oligonucleotide that binds to the rRNA.

Embodiment 7. The method of Embodiment 6, wherein the immobilized oligonucleotide is an immobilized DNA oligonucleotide.

Embodiment 8. The method of any one of Embodiments 6 and 7, wherein the immobilized oligonucleotide is an immobilized oligonucleotide that hybridizes to the rRNA, preferably hybridizes to the immobilized oligonucleotide by Watson-Crick base pairing.

Embodiment 9. The method of any one of Embodiments 1 to 8, wherein the RNA contained in the second sample comprises an RNA comprising a poly(A) stretch of nucleotides, preferably an RNA comprising a poly(A) stretch of nucleotides at a 3' end.

Embodiment 10. The method of Embodiment 9, wherein the poly(A) tailing capturing comprises binding the RNA comprising a poly(A) stretch of nucleotides and being contained in the second sample to an immobilized oligonucleotide.

Embodiment 11. The method of Embodiment 10, wherein the RNA comprising a poly(A) stretch of nucleotides hybridizes to the immobilized oligonucleotide, preferably hybridizes to the immobilized oligonucleotide by Watson-Crick base pairing.

Embodiment 12. The method of any one of Embodiments 10 to 11, wherein the immobilized oligonucleotide is an oligonucleotide comprising a poly(T) stretch of nucleotides, preferably an oligonucleotide comprising a poly(T) stretch of nucleotides attached to a 5' end.

Embodiment 13. The method of any one of Embodiments 9 to 12, wherein the RNA comprising a poly(A) stretch of nucleotides is an mRNA.

Embodiment 14. The method of any one of Embodiments 1 to 13, wherein the first sample is a dried blood sample comprising, dried on a filter paper, the blood optionally comprising blood cells and/or the first sample is a dried blood sample comprising the blood cells dried on a filter paper.

Embodiment 15. The method of Embodiment 14 wherein the dried blood sample is a blood sample that was spotted on the filter paper and dried on the filter paper, thereby forming a dried blood spot.

Embodiment 16. The method of any one of Embodiments 14 to 15 wherein the filter paper is Whatman 903 or Ahlstrom 226.

Embodiment 17. The method of any one of Embodiments 14 to 16 wherein the filter paper is part of a filter card, wherein the filter card comprises at least one dried blood spot.

Embodiment 18. The method of any one of Embodiments 15 to 17, wherein the dried blood sample comprises at least 1 to 3 dried blood spots, preferably 2 dried blood spots.

Embodiment 19. The method of Embodiment 18, wherein each dried blood spot comprises at least 50 μl blood or

at least three blood drops.

Embodiment 20. The method of Embodiment 19, wherein the blood and the blood drops were spotted onto the filter paper and allowed to dry.

Embodiment 21. The method of any one of Embodiments 15 to 20, preferably of any one of Embodiments 19 and 20, wherein the dried blood sample comprise at least 10 to 30 dried blood spot punches, wherein a dried blood spot punch is a punch of a dried blood spot and wherein each punch has a diameter of at least 2 to 4 mm, preferably at least 3 mm.

Embodiment 22. The method of any one of Embodiments 15 to 21 preferably of any one of Embodiments 19 and 20, wherein the dried blood sample comprise at least 20 dried blood spot punches, wherein a dried blood spot punch is a punch of a dried blood spot and wherein each punch has a diameter of at least 3 mm.

Embodiment 23. The method of any one of Embodiments 14 to 22, wherein the dried blood spot was stored at room temperature 30 days or less prior to extracting the RNA therefrom, preferably the dried blood spot was stored at room temperature 25 days or less prior to extracting the RNA therefrom, more preferably the dried blood sport was stored at room temperature 20 days or less prior to extracting the RNA therefrom.

Embodiment 24. The method of any one of Embodiments 1 to 23, wherein the first sample is a sample from a subject, preferably a human subject.

Embodiment 25. The method of Embodiment 24, wherein the subject is a subject suffering from a disease or a subject who is assumed to be suffering from a disease.

Embodiment 26. The method of any one of Embodiments 1 to 25, wherein the blood optionally comprising blood cells is blood of a whole blood sample and/or the blood cells are blood cells of a whole blood sample.

Embodiment 27. The method of Embodiment 26 wherein the whole blood sample is a venous blood sample or a capillary blood sample.

Embodiment 28. The method of Embodiment 27, wherein the capillary blood sample was taken from a finger of the subject, preferably by use of a finger-prick device.

Embodiment 29. The method of Embodiment 28, wherein the capillary blood was dropped from the finger onto the filter paper.

Embodiment 30. The method of any one of Embodiments 27 to 29 wherein the venous blood sample, prior to spotting the venous blood sample onto the filter paper, was stored in a tube, preferably in a tube with blood stabilizing agents.

Embodiment 31. The method of any one of Embodiments 27 to 30 wherein the venous blood sample, prior to spotting the venous blood sample onto the filter paper, was stored in a tube in the presence of EDTA, heparin tubes or acid citrate dextrose.

Embodiment 32. The method of any one of Embodiments 27 to 31, wherein the venous blood sample, prior to spotting the venous blood sample onto the filter paper, is stored in a PAXgene™ Blood tube or a Tempus™ Blood RNA Tube.

Embodiment 33. The method of any one of Embodiments 31 to 32, wherein the venous blood sample stored in the presence of EDTA was stored at -20° C after blood sampling from the subject and prior to spotting the venous blood sample onto the filter paper.

Embodiment 34. The method of any one of Embodiments 1 to 33 wherein lysing the blood cells optionally comprised by the blood and/or lysing the blood cells comprises the use of a method selected from the group comprising lysing by protein digestion, lysing by an agitation method, lysing by a homogenization method and any combination thereof.

Embodiment 35. The method of Embodiment 34, wherein lysing the blood cells optionally comprised by the blood and/or lysing the blood cells comprises lysing by protein digestion, wherein protein digestion is effected by a protease, preferably a proteinase, more preferably the protease is proteinase K.

Embodiment 36. The method of any one of Embodiments 1 to 35 wherein digesting protein contained in the first sample and/or digesting protein released from the blood cells optionally comprised by the blood and/or protein released from the blood cells is effected by a protease, preferably by a proteinase, more preferably by proteinase K.

Embodiment 37. The method of any one of Embodiments 1 to 36, wherein protecting RNA from degradation is effected by inhibiting RNAases or by inhibiting RNAses and DNAases, preferably by inhibiting RNAses and DNAases.

Embodiment 38. The method of any one of Embodiments 1 to 37, wherein the method comprises purifying the RNA.

Embodiment 39. The method of Embodiment 38, wherein the method comprises purifying the RNA contained in the second sample.

Embodiment 40. The method of Embodiment 38, wherein the method comprises purifying the RNA contained in the RNA preparation.

Embodiment 41. The method of any one of Embodiments 38 to 40 wherein the RNA is purified by using an RNA binding column.

Embodiment 42. The method of any one of Embodiments 1 to 41 wherein the method further comprises

- preparing an RNA library from the RNA preparation.

Embodiment 43. The method of any one of Embodiments 1 to 42, wherein the RNA preparation is characterized by an RNA integrity number (RIN) >2, preferably an RNA integrity number (RIN) >2.5, more preferably an RNA integrity number (RIN) >3.

Embodiment 44. The method of any one of Embodiments 1 to 43, wherein for preparing an RNA library from the RNA preparation at least 10 ng RNA of the RNA preparation are used, preferably at least 20 ng RNA of the RNA preparation are used, and more preferably at least 25 ng RNA of the RNA preparation are used.

Embodiment 45. The method of any one of Embodiments 1 to 44, wherein the RNA preparation is a liquid RNA preparation, preferably an aqueous RNA preparation, more preferably a buffered aqueous RNA preparation.

Embodiment 46. The method of any one of Embodiments 42 to 45, wherein preparing an RNA library from the RNA preparation comprises fragmenting the RNA of the RNA preparation.

Embodiment 47. The method of any one of Embodiments 1 to 47, wherein the method does not comprise amplifying the RNA extracted from the blood cells optionally comprised by the blood and/or the blood cells and/or does not comprise amplifying RNA contained in the RNA preparation.

Embodiment 48. The method of any one of Embodiments 1 to 47, wherein the RNA preparation comprises non-viral RNA.

Embodiment 49. A method of sequencing an RNA molecule in a sample, wherein the method comprises determining the nucleotide sequence of the RNA molecule and the sample is an RNA preparation prepared by a method as defined in any one of Embodiments 1 to 48.

Embodiment 50. The method of Embodiment 49, wherein determining the nucleotide sequence of the RNA molecule comprises next-generation sequencing of the RNA molecule.

Embodiment 51. A method for quantifying an RNA molecule in a sample, wherein the method comprises determining an amount of the RNA molecule in the sample by means of quantitative polymerase chain reaction or next-generating sequencing, and wherein the sample is an RNA preparation prepared by a method as defined in any one of Embodiments 1 to 48. Embodiment 52. A method for determining the course of a disease in a subject, wherein the method comprises determining an amount of an RNA molecule in a sample from the subject at various points in time, wherein the RNA molecule is a prognostic marker for the disease and the sample is an RNA preparation prepared by a method as defined in any one of Embodiments 1 to 48.

Embodiment 53. The method of Embodiment 52, wherein an amount of an RNA molecule in a sample from the subject is determined at a first point in time and at least a second point in time.

Embodiment 54. The method of any one of Embodiments 52 to 53, wherein an amount of an RNA molecule in a sample from the subject is determined at a first point in time, a second point in time and further points in time.

Embodiment 55. The method of any one of Embodiments 52 to 54, wherein the points in time are separated from each other by regular intervals.

Embodiment 56. The method of Embodiment 55, wherein the regular interval is selected from the group comprising one week, two weeks, three weeks, four weeks, one month, two months, three months, four months, five months, six months, twelve months and any multiple thereof.

Embodiment 57. The method of any one of Embodiments 52 to 54, wherein the points in time are separated from each other by irregular time intervals.

Embodiment 58. The method of any one of Embodiments 52 to 54, wherein some of the points in time are separated from each other by regular time intervals and other points in time are separated from each other by irregular time intervals.

Embodiment 59. A method for monitoring an effect of a therapy administered to a subject suffering from a disease, wherein the method comprise determining an amount of an RNA molecule in a sample from the subject after the therapy was administered to the subject and at several points in time thereafter, wherein the amount of the RNA molecule is indicative of the effect of the therapy and wherein the sample is an RNA preparation prepared by a method as defined in any one of Embodiments 1 to 48.

Embodiment 60. The method of Embodiment 59, wherein an amount of the RNA molecule in a sample from the subject is determined prior to the administration of the therapy.

Embodiment 61. The method of any one of Embodiments 59 and 60, wherein a therapy is repeatedly administered to the subject and wherein an amount of the RNA in a sample from the subject is determined after at least one of the repeated administrations of the therapy.

Embodiment 62. The method of Embodiment 61, wherein an amount of the RNA in a sample from the subject is determined after each administration of the therapy.

Embodiment 63. The method of Embodiment 62, wherein an amount of the RNA in a sample from the subject is determined at several points in time after administration each administration of the therapy.

Embodiment 64. The method of any one of Embodiments 59 to 63, wherein the points in time are separated from each other by regular intervals.

Embodiment 65. The method of Embodiment 64, wherein the regular interval is selected from the group comprising one week, two weeks, three weeks, four weeks, one month, two months, three months, four months, five months, six months, twelve months and any multiple thereof.

Embodiment 66. The method of any one of Embodiments 59 to 63, wherein the points in time are separated from each other by irregular time intervals.

Embodiment 67. The method of any one of Embodiments 59 to 66, wherein some of the points in time are separated from each other by regular time intervals and other points in time are separated from each other by irregular time intervals.

Embodiment 68. A method for determining whether a subject suffering from a disease is likely to respond to a treatment, wherein the method comprises detecting an RNA molecule in a sample from the subject, wherein the RNA molecule is a predictive marker for the disease and the sample is an RNA preparation prepared by a method as defined in any one of Embodiments 1 to 48.

Embodiment 69. The method of Embodiment 68, wherein the method comprises quantifying the RNA molecule in a sample from the subject.

Embodiment 70. A method for determining the presence or absence of a splice variant of a gene in a sample, wherein the splice variant comprises a splice junction,

wherein the method comprises determining whether the splice junction is present and if the splice junction is present, the splice variant is present and if the splice junction is absent, the splice variant is absent, and

wherein the sample is an RNA preparation prepared by a method as defined in any one of Embodiments 1 to 48.

Embodiment 71. The method of Embodiment 70, wherein determining whether the splice junction is present comprises sequencing a stretch of nucleotides suspected of comprising the splice junction.

Embodiment 72. The method of Embodiment 70, wherein determining whether the splice junction is present comprise hybridizing a probe to a stretch of nucleotides complementary to a stretch of nucleotides suspected of comprising the splice junction.

Embodiment 73. The method of any one of Embodiments 70 to 72, wherein the blood cells contained in the first sample are blood cells from a subject, wherein the subject is suffering from a disease.

Embodiment 74. A method for determining a relative abundance of an individual RNA molecule in a sample compared to the abundance of a plurality of RNA molecules in a sample, wherein the method comprises

- determining an amount of the individual RNA in a sample, wherein the sample is an RNA preparation prepared by a method as defined in any one of Embodiments 1 to 48, and

- determining the relative amount of the individual RNA in a sample to the amount of at least one RNA molecule of the plurality of RNA molecules in a sample.

Embodiment 75. The method of Embodiment 74, wherein the amount of at least one RNA molecule of the plurality of RNA molecules is retrieved from a databank.

Embodiment 76. The method of any one of Embodiments 74 to 75, wherein the amount of each RNA molecule of the plurality of RNA molecules is retrieved from a databank.

Embodiment 77. The method of Embodiment 74, wherein the amount of at least one RNA molecule of the plurality of RNA molecules in a sample is determined wherein the sample is an RNA preparation prepared by a method as defined in any one of Embodiments 1 to 48.

Embodiment 78. The method of Embodiment 74, wherein the amount of each RNA molecule of the plurality of RNA molecules in a sample is determined wherein the sample is an RNA preparation prepared by a method as defined in any one of Embodiments 1 to 48.

Embodiment 79. The method of any one of Embodiments 74 and 77 to 78, wherein the amount of the individual RNA in a sample and/or the amount of at least one RNA molecule of the plurality of RNA molecules in a sample is determined by quantitative polymerase chain reaction or next-generation sequencing.

Embodiment 80. The method of any one of Embodiments 74 to 79, wherein the individual RNA molecule is a transcript of a gene which is part of a first pathway and wherein at least one RNA molecule of the plurality of RNA molecules is a transcript of a gene which is part of a second pathway.

Embodiment 81. The method of Embodiment 80, wherein the first pathway is different from the second pathway.

Embodiment 82. The method of any one of Embodiments 74 to 81, wherein each RNA molecule of the plurality of RNA molecules is a transcript of a gene which is part of a pathway different from the first pathway.

Embodiment 83. A method for determining allele-specific expression of a gene in a plurality of samples, wherein a plurality of alleles of the gene exist, wherein the method comprises

- determining the nucleotide sequence of an RNA transcript of the gene in each and any sample of the plurality of samples,

- allocating each nucleotide sequence to an allele of the plurality of alleles of the gene, and

- calculating based on such allocation the relative abundance of an allele relative a different allele of the gene and/or to the plurality of alleles of the gene, and

wherein the sample is an RNA preparation prepared by a method as defined in any one of Embodiments 1 to 48.

Embodiment 84. The method of Embodiment 83, wherein each sample of the plurality of samples is a sample from a distinct subject.

Embodiment 85. The method of Embodiment 84, wherein the plurality of samples comprises samples from healthy subjects, subjects suffering from a disease or being at risk of suffering from a diseases, or a combination of samples from healthy subjects and subjects suffering from a disease or being at risk of suffering from a disease.

Embodiment 86. The method of Embodiment 85, wherein the disease is related to, caused by or associated with the gene.

Embodiment 87. A method for determining the proportion of subjects of a group of subjects carrying an allele of a gene that also expresses an associated trait, wherein a plurality of alleles of the gene exist, wherein the method comprises

- determining the nucleotide sequence of an RNA transcript of the gene in a sample from each and any subject of the group of subjects,

- allocating each nucleotide sequence to an allele of the plurality of alleles of the gene, and

- calculating based on such allocation the relative proportion of subjects of the group of subjects that expresses the associated trait, and

wherein the sample is an RNA preparation prepared by a method as defined in any one of Embodiments 1 to 48.

Embodiment 88. The method of Embodiment 87, wherein the associated trait causes or is associated with a phenotypic trait or a clinical symptom.

Embodiment 89. The method of any one of Embodiments 1 to 88, wherein the group of subjects comprises healthy subjects, subjects showing the clinical symptom, or a combination of healthy subjects and subjects showing the clinical symptom, preferably a combination of healthy subjects and subjects showing the clinical symptom.

Embodiment 90. The method of any one of Embodiments 87 to 89, wherein the gene is a disease related gene.

More specifically, these and other problems are solved in a first aspect by a method for preparing an RNA preparation, wherein the method comprises

- extracting an RNA from blood cells contained in a first sample, wherein the RNA extracted from the cells is provided as a second sample, and
- depleting an RNA from the second sample, wherein the RNA obtained after depleting the RNA from the second sample, is provided as the RNA preparation,

wherein

the first sample is a dried blood sample comprising said blood cells,

extracting the RNA from said blood cells comprises lysing said blood cells, digesting protein contained in the first sample and/or protein released from said blood cell, and protecting from degradation RNA released from said blood cells, and

depletion of the RNA from the second sample comprises depletion of hemoglobin RNA, depletion of rRNA and/or capturing of RNA by poly(A) tail capturing.

More specifically, these and other problems are solved in a second aspect by a method of sequencing an RNA molecule in a sample, wherein the method comprises determining the nucleotide sequence of the RNA molecule and the sample is an RNA preparation prepared by a method as defined according to the first aspect, including any embodiment thereof.

More specifically, these and other problems are solved in a third aspect by a method for quantifying an RNA molecule in a sample, wherein the method comprises determining an amount of the RNA molecule in the sample by means of quantitative polymerase chain reaction, and wherein the sample is an RNA preparation prepared by a method as defined according to the first aspect, including any embodiment thereof.

More specifically, these and other problems are solved in a fourth aspect by a method for determining the course of a disease in a subject, wherein the method comprises determining an amount of an RNA molecule in a sample from the subject at various points in time, wherein the RNA molecule is a prognostic marker for the disease and the sample is an RNA preparation prepared by a method as defined according to the first aspect, including any embodiment thereof.

More specifically, these and other problems are solved in a fifth aspect by a method for monitoring an effect of a therapy administered to a subject suffering from a disease, wherein the method comprise determining an amount of an RNA molecule in a sample from the subject after the therapy was administered to the subject and at several points in time thereafter, wherein the amount of the RNA molecule is indicative of the effect of the therapy and wherein the sample is an RNA preparation prepared by a method as defined according to the first aspect, including any embodiment thereof.

More specifically, these and other problems are solved in a sixth aspect by a method for determining whether a subject suffering from a disease is likely to respond to a treatment, wherein the method comprises detecting an RNA molecule in a sample from the subject, wherein the RNA molecule is a predictive marker for the disease and the sample is an RNA preparation prepared by a method as defined according to the first aspect, including any embodiment thereof.

More specifically, these and other problems are solved in a seventh aspect by a method for determining the presence or absence of a splice variant of a gene in a sample, wherein the splice variant comprises a splice junction,

wherein the method comprises determining whether the splice junction is present and if the splice junction is present, the splice variant is present and if the splice junction is absent, the splice variant is absent, and

wherein the sample is an RNA preparation prepared by a method as defined according to the first aspect, including any embodiment thereof.

More specifically, these and other problems are solved in an eight aspect by a method for determining a relative abundance of an individual RNA molecule in a sample compared to the abundance of a plurality of RNA molecules in a sample, wherein the method comprises

- determining an amount of the individual RNA in a sample, wherein the sample is an RNA preparation prepared by a method as defined according to the first aspect, including any embodiment thereof, and

- determining the relative amount of the individual RNA in a sample to the amount of at least one RNA molecule of the plurality of RNA molecules in a sample.

More specifically, these and other problems are solved in a ninth aspect by a method for determining allele-specific expression of a gene in a plurality of samples, wherein a plurality of alleles of the gene exists, wherein the method comprises

- determining the nucleotide sequence of an RNA transcript of the gene in each and any sample of the plurality of samples,

- allocating each nucleotide sequence to an allele of the plurality of alleles of the gene, and

- calculating based on such allocation the relative abundance of an allele relative a different allele of the gene and/or to the plurality of alleles of the gene, and

wherein the sample is an RNA preparation prepared by a method as defined according to the first aspect, including any embodiment thereof.

More specifically, these and other problems are solved in a tenth aspect by a method for determining the proportion of subjects of a group of subjects carrying an allele of a gene that also expresses an associated trait, wherein a plurality of alleles of the gene exist, wherein the method comprises

- determining the nucleotide sequence of an RNA transcript of the gene in a sample from each and any subject of the group of subjects,

- allocating each nucleotide sequence to an allele of the plurality of alleles of the gene, and

- calculating based on such allocation the relative proportion of subjects of the group of subjects that expresses the associated trait, and

wherein the sample is an RNA preparation prepared by a method as defined according to the first aspect, including any embodiment thereof.

It will be acknowledged by a person skilled in the art that the method according to the first aspect of the present invention, including any embodiment thereof, can form part of any method subject to the second, third, fourth, fifth, sixth, seventh, eighth, ninth and tenth aspect of the present invention, including any embodiment thereof. In other words, any method subject to the second, third, fourth, fifth, sixth, seventh, eighth, ninth and tenth aspect of the present invention, including any embodiment thereof, may, in an embodiment, encompass the method according to the first aspect, including any embodiment thereof, and thus the steps subject to the method according to the first aspect, including any embodiment thereof.

Without wishing to be bound by any theory, the present inventors have surprisingly found that by applying the method according to the first aspect an RNA preparation can be provided which, starting from a blood sample, provides a sufficient amount of RNA, in particular mRNA, with sufficient quality which allows the use of such RNA preparation in RNA-Seq and, therefore, the use of such RNA preparation in any method where RNA is subject to, among others, sequencing or detection. These surprising and unexpected findings apply also and in particular to a dried blood sample and allow an easily collecting, storing and transporting of a blood sample without going to the detriment of the RNA preparation prepared from such blood sample.

The term "sample" as used herein means preferably a limited quantity of a subject's material, wherein said subject's material is part of or has been taken from a subject and/or a subject's body. Preferably, said material is selected from the group comprising body fluids such as blood, a blood product, urine, saliva, cerebrospinal fluid and lymph, as well as stool or any kind of tissue and or cell material being part of a subject and/or a subject's body. It will be acknowledged by a person skilled in the art that the presence of and/or an RNA in said sample is intended to be similar to and represent the presence and/or the level of the RNA in a larger amount of that subject's material. More precisely and as an illustrative, non-limiting example, a level of the RNA determined in a sample of, e.g., some ml of blood from a subject also represents a level of said RNA in the blood of the subject's body. Furthermore, in an embodiment of the methods of each and any aspect of the invention, a sample from the subject comprises said subject's material in a form, for example processed, fixed and/or preserved such that said sample is suitable for use in the methods of each and any aspect of the invention. The subject's material in the sample may thus be diluted, for example with a solvent suitable for the method of each and any aspect of the invention such as methanol and/or water, may be dried, for example on a filter card, may be resolved after having been dried such, for example with a solvent suitable for the method of the invention such as methanol and/or water, or a substance may be added, wherein said substance prevents blood from coagulation such as for example EDTA, heparin or citrate. Such substances are referred herein as blood stabilizing agents. Specific tubes for storage of RNA (stabilizing the RNA) are the so calles PAXGene™ Blood Tubes and the Tempus™ Blood RNA tubes. Blood samples collected in a tube with EDTA are referred herein as EDTA blood sample.

A sample as preferably used in connection with each and any aspect of the present invention a sample as used in such methods is prepared from a primary source such as whole blood. Other samples include, but are not limited to, serum samples and plasma samples. As a preferred embodiment of the invention whole blood is venous blood or capillary blood. Venous blood is taken from the vein by taking a blood sample. Capillary blood taken from the finger by taking a blood sample by use of a finger prick.

Whole blood comprises blood cells. Blood contains many types of blood cells: white blood cells (such as monocytes, lymphocytes, neutrophils, eosinophils, basophils, and macrophages), red blood cells (erythrocytes), and platelets. Blood cells according to the present invention are all of the these cells (white blood cells [such as monocytes, lymphocytes, neutrophils, eosinophils, basophils, and macrophages], red blood cells [erythrocytes], and platelets).

In an embodiment of the various aspects of the invention the primary sample is whole blood which is, in an embodiment, processed such that it is collected on a dry blood filter card (also referred herein as dried blood filter card or filter card). Such sample is also referred to herein either as a dry blood filter sample, dried blood filter sample, a dry blood spot on a filter card or a dried blood spot on a filter card. Dried blood filter cards comprise an area consisting of filter paper where the blood can be dripped on, whereby the place where the blood should be dripped on is marked by a circle. Dependent of blood volume dripped on the filter paper the spot can have a different size. Such a spot can have circle area of 190 mm2, e.g. if 100 µl of EDTA blood was dripped on the filter paper. Normally 50 to 100 µl of blood are dripped on a filter paper, preferably 50 to 60 µl. 50 µl to 60 µl corresponds to 3 drops of blood. A person skilled in the art will acknowledge that the exact volume thus collected may vary depending on the hematocrit of the specific patient.

The filter paper of the filter cards is manufactured from 100% pure cotton fiber with no wet strength additives. The critical physical properties during manufacturing are basis weight, ph and ash. Basis weight should 110 lb +/- 5% per ream (179 g(m3 +/- 5%). A ream is defined as 500 sheets 24" x 36" (ASTM D646-96). The pH should ne 5.7 to 7.5 (test method ISO 6599:1981). The ash should have a maximum of 0.1% (test method A o ASTM D586-97a). Filter papers that meet the properties are Ahlstrom 226 filter paper/ specimen collection paper (also referred to as Ahlstrom Grade 226 filter paper) and Whatman® Grade 903 filter paper/ Whatman Body Fluid Collection Paper (Whatman BFC 18. K932661).

A specific design or type of a filter card is the CentoCard®. Beside parts that are needed for information and for ordering test, the CentoCard® comprises like other filter cards an area consisting of filter paper where the blood can be dripped on, whereby the place where the blood should be dripped on is marked by a circle. The filter paper of the CentoCard® is the Ahlstrom 226 filter paper/ specimen collection paper.

Ribonucleic acid (RNA) occurs in different forms within organisms and serves many different role. RNAs involved in protein synthesis are messenger RNA(mRNA), ribosomal RNA (rRNA), signal recognition particle RNA (7SL RNA or SRP RNA), Transfer RNA

| RNAs involved in protein synthesis are **Type** | **Abbr.** | **Distribution** |
|---|---|---|
| Messenger RNA | mRNA | All organisms |
| Ribosomal RNA | rRNA | All organisms |
| Signal recognition particle RNA | 7SL RNA or SRP RNA | All organisms |
| Transfer RNA | tRNA | All organisms |
| Transfer-messanger RNA | tmRNA | Bacteria |

RNAs involved in post-transcriptional modification or DNA replication:

| **Type** | **Abbr.** | **Distribution** |
|---|---|---|
| Messenger RNA | mRNA | All organisms |
| Ribosomal RNA | rRNA | All organisms |
| Signal recognition particle RNA | 7SL RNA or SRP RNA | All organisms |
| Transfer RNA | tRNA | All organisms |
| Transfer-messenger RNA | tmRNA | Bacteria |

In an embodiment of the present invention and more specifically in an embodiment of the first aspect, the RNA is extracted from blood. In a preferred embodiment, blood comprises a cellular compartment, preferably comprising blood cells, and a non-cellular compartment typically comprising the parts and compartment, respectively, which is different from the cellular compartment of the blood. In a more preferred embodiment, the non-cellular compartment comprises serum and/or plasma.

In an embodiment of the present invention and more specifically in an embodiment of the first aspect, the RNA preparation is a preparation comprising an mRNA, where the content, either the absolute content or any relative content, of the mRNA is increased compared to the content of the mRNA in the blood or blood cells from which the RNA is extracted.

In an embodiment of the present invention and more specifically in an embodiment of the first aspect, the RNA preparation is an mRNA preparation, where the content, either absolute content or any relative content, of the mRNA is higher than the content of any other RNA contained in said mRNA preparation.

In an embodiment and as preferably used herein, hybridization of two nucleic acid molecules to each other, is through Watson-Crick base pairing.

In an embodiment of the present invention and more specifically in an embodiment of the first aspect, a dried blood spot is generated by spotting at least 50 μl of blood onto a filter paper typically forming part of a filter card. In an alternative embodiment of the present invention and more specifically in an embodiment of the first aspect, a dried blood spot is generated by transferring at least three blood spots from a blood sample onto a filter paper typically forming part of a filter card.

In an embodiment of the present invention and more specifically in an embodiment of the first aspect, the RNA preparation obtained by the method of the first aspect, comprises non-viral RNA such as mammalian and human RNA in particular. It will be acknowledged by a person skilled in the art that said RNA preparation, in an embodiment, comprises viral RNA and viral mRNA in particular, if such viral mRNA is contained in the blood or the blood cells (i.e., the first sample). In a preferred embodiment thereof, the content of such viral RNA and viral mRNA in particular is reduced so that its content, relative content or absolute content, is decreased compared to its content in the first sample, i.e. the blood and blood cells, respectively, from which the RNA is extracted in accordance with the present invention.

In an embodiment of the present invention and more specifically in any one of the first, second, third, fourth, fifth, sixth, seventh, eighth, ninth or tenth aspect, including any embodiment thereof, irregular time intervals are time intervals which do not follow a regular scheme and/or predictable scheme. A predictable scheme is preferably one where a distinct time interval can be predicted based on one or several time intervals preceding said distinct time interval. A typical reason for of the cause of irregular time intervals can be the decision of a physician treating a or the subject to change the treatment in light of any aggravation or improving of the health condition of the subject.

In an embodiment of the present invention and more specifically in any one of the first, second, third, fourth, fifth, sixth, seventh, eighth, ninth or tenth aspect, including any embodiment thereof, the terms allele and genetic variant are used interchangeably herein.

In an embodiment of the present invention and more specifically in any one of the first, second, third, fourth, fifth, sixth, seventh, eighth, ninth or tenth aspect, including any embodiment thereof, a predictive biomarker is a compound, such as nucleic acid molecule, that indicates sensitivity or resistance of a subject to a specific therapy.

In an embodiment of the present invention and more specifically in any one of the first, second, third, fourth, fifth, sixth, seventh, eighth, ninth or tenth aspect, including any embodiment thereof, a prognostic biomarker is a compound, such as a nucleic acid molecule, that can be used to measure the progress of a disease of a subject in a sample from the subject.

The present invention is now further illustrated by the following figures and examples from which further features, embodiments and advantages may be taken.

More specifically,

Fig. 1 shows dried blood spots on a blood filter card prepared from 100μl of EDTA blood;

Fig. 2 shows the RNA integrity [RIN = RNA integrity number] of RNAs obtained from filter cards after storage of the blood filter cards of up to 21 days at room temperature. RIN values were assessed with the RNA HS Screentape and the Agilent Tapestation;

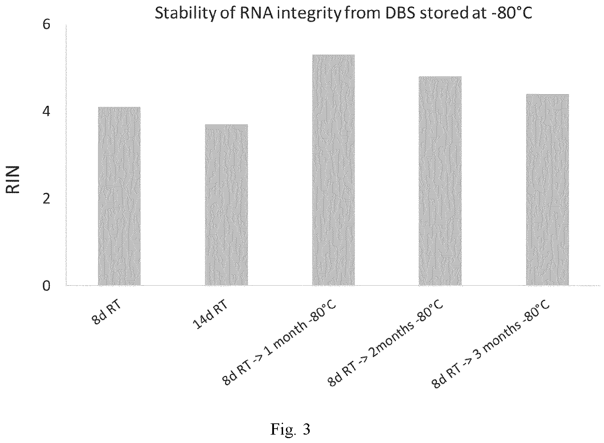

Fig. 3 shows influence of storage of blood filter cards on the RNA integrity [RIN = RNA integrity number] of RNAs obtained from blood filter cards: a) the blood filter cards were stored at room temperature for 8 days, b) the blood filter cards were stored at room temperature 14 days, c) the filter cards were first stored at room temperature for 8 days and then for one month at -80°C, d) the filter cards were first stored at room temperature for 8 days and then for two month at -80°; e) the filter cards were first stored at room temperature for 8 days and then for three months at -80°; RIN values were assessed with the RNA HS Screentape using the Agilent Tapestation;

Fig. 4 shows a boxplot showing the RNA yield [ng] of RNAs obtained from a total of 583 filter cards with a card age between 1 day and 14 days, wherein the filter cards were stored at -80°C prior to RNA extraction; RNA concentrations were assessed with the Qubit HS RNA assay;

Fig. 5 shows a boxplot showing the RNA integrity [RIN = RNA integrity number] of RNAs obtained from a total of a total of 583 filter cards with a card age between one day and 14 days; RIN values assessed with Agilent Tapestation RNA HS Screentape;

Fig. 6 shows fraction of samples of RNA obtained from filter cards with a RIN > 2.5 (dark grey) and RIN < 2.5 (light grey) over the card age [d]; the line is showing the percentage of cards with a RIN > 2.5; RIN values assessed with Agilent Tapestation RNA HS Screentape.

Fig. 7 shows gene expression correlation of (log2 corrected read counts) for RNA obtained from EDTA blood sample and DBS (same individual); the Pearson correlation coefficient is r = 0.91; the data reflects the results from a protocol version only using Poly(A)-selection and globin depletion.

Fig. 8 shows gene body coverage for RNA samples obtained from dried blood spots; the graph shows the 5' - 3' coverage of the samples, where a shift to the 3' end indicates higher RNA degradation; the data reflects the results from a protocol version only using Poly(A)-selection and globin depletion;

Fig. 9 shows gene body coverage for RNA samples obtained from dried blood spots; the graph shows the 5' - 3' coverage of the samples, where a shift to the 3' end indicates higher RNA degradation; the data presented here were obtained with the protocol presented herein (see Example 1, Example 4 and Example 6);

Fig. 10 shows gene expression correlation (log2 corrected read counts) for RNA obtained from EDTA blood sample and DBS (same individual). The Pearson correlation coefficient is r = 0.95; the data presented here were obtained with the protocol presented herein (see Example 1, Example 4 and Example 6);

Fig. 11 shows gene expression correlation (log2 corrected read counts) for RNA obtained from PAXgene blood samples and DBS for two individuals (A) and B)) ; the Pearson correlation coefficient is A) r = 0.92 B) r= 0.92, also demonstrating the high reproducibility of the method based on the protocol presented herein (see Example 1, Example 4 and Example 6);

Fig. 12 shows a Venn diagram showing the overlap between OMIM genes (genes with phenotype-causing mutation, www.omim.org) and genes with an RPKM>4 (RPKM = reads per kilobase per million) from A) RNA obtained from blood (RIN=9.1) and B) RNA obtained from DBS card (RIN=5.4).

Fig. 13 shows a Pie chart showing the results of 103 patients with a predicted/potential splice variant identified in whole exome/ whole genome sequencing that were reanalyzed by RNA seq of RNA obtained from DBS to confirm/reject an effect on the RNA transcript. *Likely abnormal splicing = <5 supporting reads;

Fig. 14 shows a Sashimi plot (IGV) showing a heterozygous variant in the DYSF gene (c.5785-824C>T); the deep intronic variant results in intron retention in form of a cryptic exon;

Fig. 15 shows a sashimi plot (IGV) showing a homozygous variant in the DNAJB2 gene (c. 176-2A>G). The canonical splice site variant leads to the skipping of Exon04 of the transcript;

Fig. 16 shows Sashimi plot (IGV) showing a heterozygous variant in the SBDS gene (c.258+2T>C) leading to an altered splice site in some of the reads;

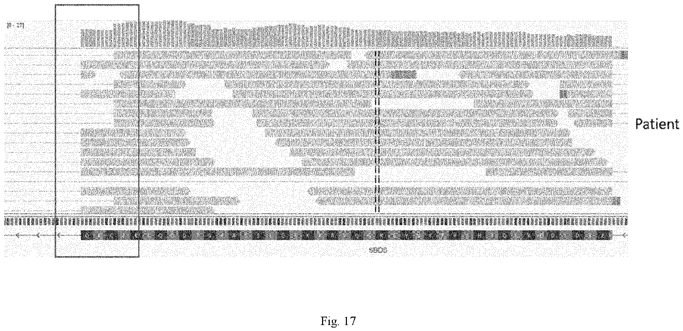

Fig. 17 shows IGV screenshot of the patient presented in Figure 15; some transcripts show a deletion of the last 8 bases in Exon 02 of the SBDS gene;

Fig. 18 shows a gene set enrichment analysis in a Parkinson disease cohort vs controls whereby a positive or negative enrichment score (ES) indicates gene set enrichment; Example A) shows the MTOR signaling pathway enriched in the patient cohort carrying a GBA variant; Example B) shows negative regulation of the lysosome pathway in the same cohort;

Fig. 19 shows a IGV screenshot showing the allele penetrance of heterozygous GBA mutation N409S wherein the example is showing a balanced allele frequency;

Fig. 20 shows a IGV screenshot showing the allele penetrance of a heterozygous GBA mutation N409S; the example is showing an imbalanced allele frequency (70:30);

Fig. 21 shows clustering results of time course analysis of RNA seq data showing upregulation of a gene cluster at timepoint Post 1 (Pre1 and Pre2 data were merged to one timepoint);

Fig. 22 shows pathway enrichment for Cluster 1 (presented in Figure 21) using ToppGene showing upregulation in the interferon alpha/beta signaling pathway;

Fig. 23 shows normalized counts (DESeq2) from DBS samples of individuals before and after covid19 vaccination (pre1:3-4 days prior vaccination, pre2: 1-2 days prior, post1: 1-2 days after vaccination, post2: 3-4 days after, post3: 5-7days after); time course analysis showed the upregulation of genes from the interferon alpha/beta pathway as an early response to the vaccine (here: EIF2AK2 and IFI35); and

Fig. 24 shows normalized counts (DESeq2) from DBS samples of individuals before and after covid19 vaccination (pre1: 3-4 days prior vaccination, pre2: 1-2 days prior, post1: 1-2 days after vaccination, post2: 3-4 days after, post3: 5-7days after); time course analysis showed the upregulation of genes from the interferon alpha/beta pathway as an early response to the vaccine (here: IRS2 and MAP4K4).

**Examples**

In the Examples described in the following a dry blood spot (abbr. DBS) on a filter card was used as a sample from a subject. The filter paper of the filter card is the Ahlstrom 226 filter paper. Specifications Ahlstrom 226 filter paper can be found within the document. Nevertheless, a person skilled in the art will acknowledge that depending on the used type of sample from a subject, e.g. comprising saliva, cerebrospinal fluid, plasma, serum, full blood, blood on a dry blood filter card or another blood product, the method of the present invention has to be adjusted to the type of sample according to the method described in the following examples.

**Example 1: RNA extraction**

| Reagents /Materials | Manufacturer (Order ID) |
|---|---|
| Quick-RNA Microprep Kit | Zymo (R1051) |
| PK Digestion Buffer | Zymo (R1200-1-20) |
| Proteinase K w/ Storage Buffer Set | Zymo (D3001-2-125) |
| Nuclease-free water | e.g., Carl Roth |
| DNA/RNA Shield | Zymo (R1200-125) |
| Qubit RNA HS Assay Kit | Life Technologies |
| RNA High Sensitivity ScreenTape Sample Buffer | Agilent (5067-5577) |
| RNA High Sensitivity ScreenTape | Agilent (5067-5576) |
| RNA High Sensitivity ScreenTape Ladder | Agilent (5067-5578) |
| ≥99,5 % Ethanol | e.g., Carl Roth |
| 2 ml tube | e.g., Carl Roth |
| Low bind tube | e.g., Carl Roth |
| Nuclease free water | e.g., Carl Roth |

**Proteinase K Master Mix**

| Reagent | 1 reaction |
|---|---|
| 2 x DNA/RNA shield | 350 $\mu$l |
| Nuclease-free-water | 350 $\mu$l |
| PK Digestion Buffer | 70 $\mu$l |
| Proteinase K | 30 $\mu$l |
| Total | 800 $\mu$l |

**DNase1 Digestion Mix**

| Reagent | 1 reaction |
|---|---|
| Digestion Buffer | 35 $\mu$l |
| DNaseI | 5 $\mu$l |

(continued)

| Reagent | 1 reaction |
|---------|------------|
| Total | 40 µl |

**Working solution of the Qubit RNA HS assay (RNA specific fluorescent dye)**

| Reagent | 1 reaction |
|---------|------------|
| Qubit™ RNA HS Reagent | 1 µl |
| Qubit™ RNA HS Buffer | 199µl |

**Filter card & blood samples**

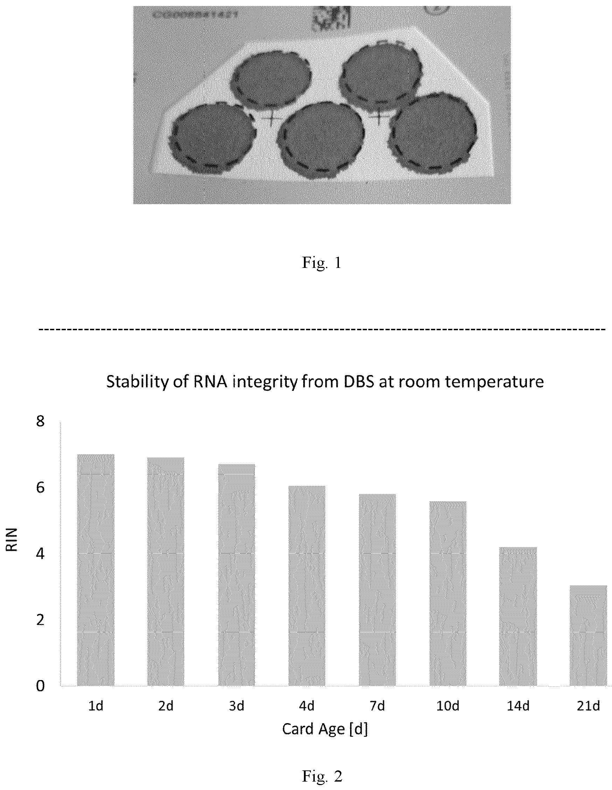

For the preparation of the dried blood samples from EDTA blood tube, a sample was collected using the Vacutainer™ Plastic K2EDTA (BD). The tube was inverted 10 times to ensure mixing of EDTA and blood. 50 to 100µl of blood were dropped onto each spot of the filter card (Figure 1). The cards were dried open for 2 hours at room temperature. Afterwards the filter cards were kept in a plastic envelope at room temperature until further processing. How the blood was collected and which amount of blood was spotted on the filter is specified in each example.

**Extraction protocol**

Two complete spots (circle area: 2 x 95 = 190 mm$^2$) were cut from filter card (or 20 punches with 3mm (circle area: $20 \times 7{,}07$ mm$^2$ = 141,40 mm$^2$) for each patient and placed into a 2ml reaction tube. 800µl of the Proteinase K Master Mix were added to the 2 ml reaction tube and the reaction tube was incubated on a thermos shaker for 30min at 37°C and 1500rpm. 450µl of the supernatant was transferred to a new 2ml tube, 450µl of 100% ethanol was added and mixed. The mixture was centrifuged. 450µl of the supernatant was transferred to an RNA Column (Zymo Spin Column) that was placed on a collection cube and centrifuged 30 sec at 16.000 rcf. The flow through was discarded, and again 450µl of the supernatant was transferred to the RNA Column (Zymo Spin Column, Zymo R1051) that was placed on a collection cube and centrifuged 30 sec at 16.000 rcf. The flow throw was discarded and 400 µl RNA Wash Buffer (Zymo R1051) was added to the column, then tube with column centrifuged for 30 sec at 16.000 rcf and the flow through discarded. 40µl DNase1 Digestion Mix was added to the column and incubated for 15 min at room temperature. Afterwards 400µl Prep Buffer (Zymo R1051) was added to the column, centrifuged for 30 sec at 16.000 rcf and flow through discarded. 700µl RNA Wash Buffer were added to the column, centrifuged for 30 sec at 16.000 rcf and flow through was discarded. 400µl RNA Wash Buffer was added to the column, centrifuged for 2 min at 16.000 rcf and flow through discarded. The tube was centrifuged again to remove every liquid from the column. Afterwards the column was transferred on a fresh low bind tube, 15 µl nuclease free water was added, incubated one minute at room temperature, centrifuged 30 sec at 16.000 rcf, the column discarded, and the sample stored on a cooling rack.

**RNA quality control**

2µl of the sample was combined with 198µl of working solution of the Qubit RNA HS assay dye to measure the RNA concentration (uses fluorescent dye specific for RNA) on a Qubit device (Life Technologies).
1 µl RNA High Sensitivity buffer was mixed with 2 µl sample. The mixture was incubated 3 minutes at 72°C and run with an RNA High Sensitivity Screen tape on the Agilent Tapestation to determine the RNA integrity number (RIN).

**Optimization of the protocol** - **Proteinase K Master Mix**

The Proteinase K Master Mix contains a DNAse and RNAse inhibitor, e.g. DNA/RNA shield (Zymo Research) and proteinase K to lyse the cells and inactivate RNAses to prevent RNA degradation.
Samples 1-3 were incubated according to the described protocol (Example 1) with Proteinase K Master Mix, samples 4-6 were incubated without proteinase K and proteinase K digestion buffer but only the DNA/RNA shield (volumes of proteinase K and digestion buffer replaced with DNA/RNA shield). RNA amount and RIN were significantly reduced in the 3 samples without proteinase K (see Table 1 ). Therefore, proteinase K is essential for RNA extraction from DBS.

*Table 1: RNA concentration [ng/μl] and RIN values from extractions with proteinase K (with PK mix) and without (no PK mix).*

| No | Condition | RNA concentration [ng/μl] | RIN |
|---|---|---|---|
| 1 | with PK mix ctrl 1 | 7.29 | 6.3 |
| 2 | with PK mix ctrl 2 | 6.15 | 6.4 |
| 3 | with PK mix ctrl 3 | 5.99 | 6.1 |
| 4 | No PK mix ctrl 1 | 0.86 | 2.6 |
| 5 | No PK mix ctrl 1 | 1.01 | 2.4 |
| 6 | No PK mix ctrl 1 | 0.86 | 2.5 |

**Example 2: RNA stability on filter cards**

Initial experiments were addressing the time stability of RNA from dried blood spots (EDTA blood), i.e., how much degradation and what RNA yields can be observed. The stability was tested over a period of 21 days. For each timepoint 2 spots from 2 different filter cards, prepared according to Example 1, were cut out with a scalpel and RNA was extracted. The RNA extractions for this experiment were deviating from the described protocol (see Example 1) in the sense that each spot was incubated individually in a 2ml tube at 37°C and 1500rpm with 400μl of the Proteinase-K-Mix. After the incubation and addition of 1x volume 100% ethanol to each tube the whole volume of the tubes was added stepwise to one Zymo spin column and centrifuged according to the standard protocol described in Example 1. Furthermore, the elution volume of 25μl of nuclease-free water differed from the standard protocol described in Example 1.

Figure 2 shows the results of the stability test. The RIN remained stable over the first 3 days. After 4 days the RIN was decreased to 6 and it stayed stable until day 10. After 14 days we observed a RIN of 4 and after 21 days the RIN value was further reduced to 3.

The presented data show that with the herein described protocol RNA of high quality can be extracted from dried blood spots over a period that makes it available for diagnostic settings, where shipment of filter cards often takes several days to 2 weeks.

We also examined the stability of RNA from DBS samples that were frozen at -80°C for 1 month, 2 months and 3 months, respectively. For this purpose, 2 filter cards were prepared according to the description in Example 1 and kept at room temperature for 8 days. After 8 days 12 spots from the filter cards were cut out with a scalpel. For each timepoint (1, 2 and 3 month(s)) 4 spots were stored in a 2ml tube at -80°C. After the respective times RNA was extracted from these spots.

Additionally, RNA was extracted from 4 spots of the same filter cards after 8 days and 14 days at room temperature. The RNA extractions for this experiment were deviating from the described protocol (see Example 1) in the sense that 4 spots were used where each spot was incubated individually in a 2ml tube at 37°C and 1500rpm with 400μl of the Proteinase-K-Mix. After the incubation and addition of 1x volume 100% ethanol to each tube the whole volume of the tubes was added stepwise to one Zymo spin column and centrifuged according to the standard protocol described in Example 1. Furthermore, the elution volume of 25μl of nuclease-free water differed from the standard protocol described in Example 1.

Figure 3 shows the results of the stability at -80°C experiment. During the 3 months of storage at -80°C the RIN value of the sample showed only a slight decrease from 5.3 to 4.4. The storage of the filter cards at -80°C prevented further degradation of the RNA on the filter card and the integrity could also be preserved during the extraction procedure.

The presented data show that with the herein described protocol RNA of high quality can be extracted from dried blood spots over a period that makes it available for diagnostic settings, for example for samples that were stored for several months at -80°C.

**Example 3: Characteristics of extracted RNAs from real-life samples**

RNA from dried blood spots of consented patients was extracted to investigate the usability of RNA from DBS in a real-life diagnostic setting. In these cases the physicians collected venous blood from their patients, transferred the blood to EDTA sample tubes and dropped 50 μl of the EDTA blood on each spot of the blood filter card.

Therefore, the filter cards were collected and stored at -80°C after the arrival at Centogene. Filter cards with a card age ≤14 days (card age = time [d] of filter card preparation until arrival in the laboratory) went into RNA extraction following the protocol described in Example 1.

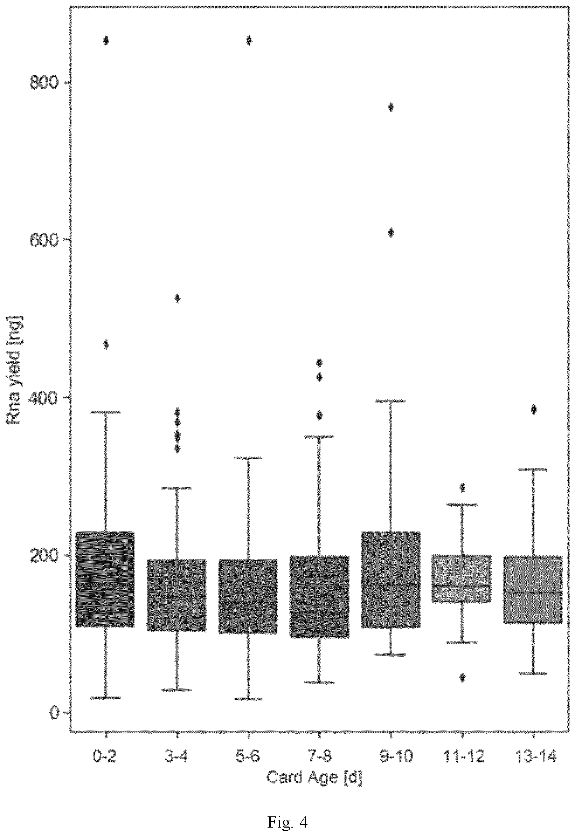

Extracted RNAs showed the following characteristics:
As presented in Figure 4, the RNA yield of 583 DBS samples was stable over the examination period of 14 days. For a card age up to 14 days it was shown that RNA yield for more than 99% of the cards was more than 25 ng after extraction which according to the extraction protocol described herein (see Example 1), was tested as the minimum required amount for RNA library preparation (see Example 5).

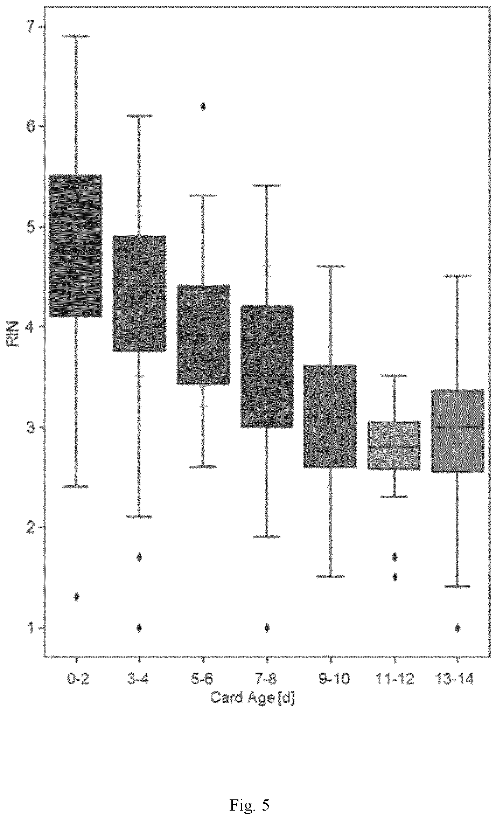

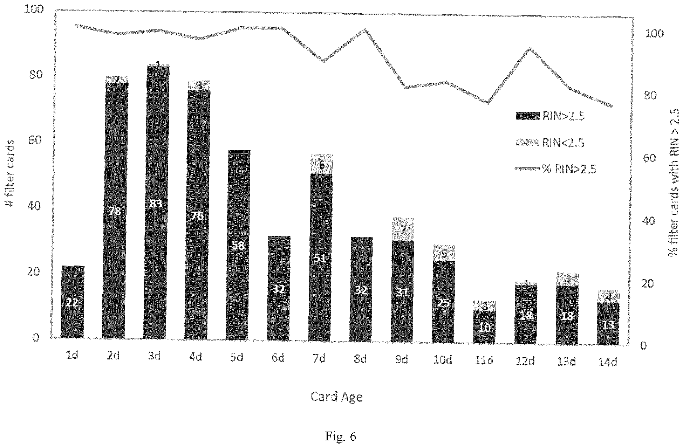

As shown in Figure 5, the RNA integrity got compromised over time. However, over the first 7 days more than 97% of the extracted filter cards had a RIN value above 2.5. After 7 days up to 14 days, this was still true for 85% of the filter cards (see Figure 6).

In our initial experiments samples with a RIN value below 2.5 showed a high probability of failing during sequencing, e.g., low number of unique assigned reads. Therefore, we have defined 2.5 as a cutoff for the RNA integrity.

The presented data show that with the herein described protocol RNA of high quality can be extracted from dried blood spots over a period that makes it available for diagnostic settings, where shipment of filter cards often takes several days to 2 weeks.

It was also tested if the same RNA quality can be achieved from finger prick samples. Therefore, up to 6 blood drops were put onto one filter card spot with 2 spots in total. The filter cards were dried for 24 hours at room temperature. Afterwards extraction was performed according to Example 1. Table 1 shows the results of the extraction. Due the good RNA quality of the finger prick samples an RNA library was generated as described in Example 4. Afterwards the sample was sequenced (see Example 6). Sequencing results were compatible with results from the EDTA blood filter cards. This allows a more convenient way of sample collection, especially for repeated sampling as in longitudinal studies.

Table 2: RIN and RNA amount extracted from 2 dried blood spots prepared from finger prick of 3 individuals.

| Sample | RIN | RNA yield [ng] |
|---|---|---|
| Prick Test 1 | 6.5 | 39.6 |
| Prick Test 2 | 6.5 | 81 |
| Prick Test 3 | 6.2 | 126 |

**Example 4: Library Preparation protocol**

| Reagents | Manufacturer (Order ID) |
|---|---|
| Polaris Depletion Kit - rRNA/Globin (HMR) Box 1/2 | Watchmaker Genomics (9K0077-096) |
| Watchmaker RNA Library Prep Kit Box 2/2 | Watchmaker Genomics (9K0078-096) |
| Nuclease-free water | e.g. Carl Roth |
| D1000 ScreenTape | Agilent (5067-5582) |
| D1000 Reagents | Agilent (5067-5583) |
| Qubit dsDNA HS Assay Kit, 500 | Thermo Fisher Scientific |
| Agencourt AMPure XP | Beckman Coulter |
| ≥99,5 % Ethanol | e.g., Carl Roth |
| xGen Stubby Adapter-UDI Primers | IDT (10005921) |
| Poly(A) RNA Selection Kit V1.5 | Lexogen (157.96) |
| Quantabio sparQ PureMag beads (alternative: RNA clean XP beads) | VWR (733-2627) (alternative: Beckman-Coulter (A63987)) |
| ERCC control | ThermoFisher (4456740) |

**Poly(A) selection**

Preparation of Poly(A) selection beads

2 μl Poly(A)-selection beads (Lexogen) per sample were transferred into a 1.5ml tube, sedimented by a magnet until the supernatant was clear. The supernatant was removed and discarded. The tube was removed from the magnet and 75 μl Bead Wash Buffer (BWB, Lexogen Poly(A) selection kit) per Poly(A) selection sample was added. The washing step was repeated two times for a total of 2 washes and the Bead Wash Buffer (BWB, Lexogen Poly(A) selection kit) was removed after the last washing step. Afterwards the beads were resuspended in 10μl Hybridization Buffer (HYB, Lexogen Poly(A) selection kit) per samples.

Denaturing the RNA

25ng - 50ng total RNA was diluted in nuclease-free water to a volume of 10μl and incubated in a thermocycler at 60°C for 1 min. Until the beads were added the sample was kept at 25°C.

Hybridizing Poly(A) RNA

10μl of denatured RNA was added to 10μl of washed beads and incubated in a thermomixer at 25°C for 20 min with 1250rpm. The plate was transferred onto a magnetic rack and incubated until the supernatant was clear and was removed. The plate was removed from the rack and 100μl BWB was added. The beads were resuspended and incubated on the thermomixer at 25°C for 5 min with 1250rpm. Afterwards the beads were collected on the magnet until the supernatant was clear and removed. The washing step were repeated for a total 2 washes and the end the supernatant was completely removed.

RNA Elution

The RNA was eluted from the Poly(A) beads with 20 μl nuclease free water and incubation at 70°C for 1 min. The plate was immediately placed on the magnet until the supernatant was clear. 18μl of the supernatant was transferred to the corresponding well of a new 96 well PCR plate for further processing.

**rRNA and hemoglobin RNA depletion**

rRNA and Globin Depletion

For each reaction, the Depletion Reaction Mix was prepared (on ice).

| Component | Volume (μl) |
|---|---|
| Depletion Master Mix | 4.5 |
| Depletion Probes - rRNA/Globin (HMR) | 2.5 |

7μl of Depletion Reaction Mix were added to the eluted RNA on a plate, mixed and the sealed plate was centrifuged. The plate was placed on a thermal cycler and the following Depletion program:

| Step | Temperature | Time |
|---|---|---|
| Hold | 77°C | Hold |
| Denaturation | 77°C | 2 min |
| rRNA Digestion | 65°C | 15 min |
| Hold | 4°C | Hold |
| Heated lead: 80°C | Volume: 25 μl | |

DNase Digestion

The sealed PCR plate was centrifuged and 35μl of DNase Master Mix was added to the depleted RNA. The sealed plate was vortexed, centrifuged and placed on a thermal cycler to run the following DNase Digestion Program.

| Step | Temperature | Time |
|---|---|---|
| Pre-cooling | 4°C | Hold |
| DNase I digestion | 37°C | 10 min |

(continued)

| Step | Temperature | Time |
|---|---|---|
| Hold | 4°C | Hold |
| Heated lead: 40°C | Volume: 60μl | |

Post-digestion Cleanup

108 μl (1.8X) of sparQ PureMag beads (Quantabio) were added to each reaction. The plate was incubated for 10 min on a thermal shaker set to 20-25°C shaking at 1200 rpm. Afterwards the plate was placed on the magnetic stand until the solution was clear. Then the supernatant was removed from each well and 200μl of 80% EtOH were added to each well. After 30 secs the EtOH was removed. This washing step was repeated for a total of two washes. Afterwards the EtOH was removed, and the remaining ethanol was evaporated.

**Library Preparation**

Fragmentation and Priming

The Frag & Prime Master Mix was prepared as follows for each reaction.

| Component | Volume (μL) |
|---|---|
| Frag. & Prime Buffer | 10.8 |
| RNase free water | 16.2 |

27 μL of the Frag. & Prime Master Mix was added to the dry beads, vortexed and centrifuged.

Fragmentation program:

| Step | Temperature | Time |
|---|---|---|
| Pre-cooling | 4°C | Hold |
| RNA Fragmentation | 85°C | 3 min |
| Hold (Priming) | 12°C | Hold |
| Lid temperature: 105°C | Volume: 27μl | |

The plate was placed on the thermal cycle and the program Fragmentation program was run. After the program was finished, the plate was placed on a magnet until all beads were collected on the tube wall and the solution was clear. 25 μl of the clear supernatant containing the fragmented libraries are transferred into the corresponding well of a new 96 well plate that contains 10 μl of pre-aliquoted 1st Strand Synthesis Master Mix. After mixing the was placed in thermocycler and the 1st Strand Synthesis Program was started.

1st Strand Synthesis

1st Strand Synthesis Program

| Step | Temperature | Time |
|---|---|---|
| Pre-cooling | 4°C | Hold |
| | 25°C | 10 min |
| cDNA Synthesis | 42°C | 15 min |
| RT Inactivation | 70°C | 15 min |

(continued)

| Step | Temperature | Time |
|---|---|---|
| Hold | 4°C | Hold |
| Lid temperature: 105°C | Volume: 35µl | |

For each reaction the 1st Strand Master Mix was prepared as follows:

| Component | Volume (µl) |
|---|---|
| 1st Strand Buffer | 9 |
| 1st Strand Enzyme | 1 |

2nd Strand Synthesis & A-Tailing

2nd Strand Synthesis program

| Step | Temperature | Time |
|---|---|---|
| Pre-cooling | 4°C | Hold |
| 2nd Strand Synthesis | 42°C | 5 min |
| A-Tailing | 62°C | 10 min |
| Hold | 4°C | Hold |
| Lid temperature: 80° | Volume: 50µl | |

For each reaction, 2nd Strand Master Mix was prepared as follows:

| Component | Volume (µl) |
|---|---|
| 2nd Strand Buffer | 14 |
| 2nd Strand Enzyme | 1 |

15µl of the 2nd Strand Master Mix was added to the 1st Strand Synthesis reaction, mixed and centrifuged and placed in the thermocycler to run the 2nd Strand Synthesis program.

Adapter Ligation

NOTE: See Prior to Starting for considerations in adapter selection and design.

Adapter Ligation Program

| Step | Temperature | Time |
|---|---|---|
| Pre-cooling | 4°C | Hold |
| Ligation | 20°C | 15 min |
| Hold | 4°C | Hold |
| Lid temperature: OFF | Volume: 100µl | |

The thawed Ligation Buffer was vortexed for 20 sec to fully homogenize the solution. The truncated "stubby" adapters (IDT) are diluted from 15μM to 0.4 μM. 5μl of the diluted adapters were added to each well containing the 2nd strand mix.

For each reaction the Ligation Master Mix was prepared as follows:

| Component | Volume (μl) |
|---|---|
| Ligation Buffer | 40 |
| Ligase Enzyme | 5 |

The Ligation Master Mix was then vortexed for another 20 sec to thoroughly mix the reagents. 45 μl of the Ligation Master Mix were added to each well and mixed by pipetting for 10 x with the pipette set to 80μl. The plate was then placed in a thermocycler and the Adapter Ligation Program was started.

Once the program was finished the post ligation cleanup was performed.

Post-ligation Double Cleanup

70 μl (0.7X) of Ampure Beads (equilibrated to RT) were added to each ligation reaction, then mixed and incubated for 5 min at RT, then placed on a magnet until the solution was clear. The supernatant was removed from each well. Afterwards 200 μl of freshly prepared 80% ethanol was added to each well, incubated for 30 sec and EtOH was removed. The washing steps were repeated for a total of two washes. Then the EtOH was removed and evaporated for 5 min at RT. The plate was removed from the magnet and each bead pellet was eluted in 22 μL nuclease-free water. The plate was incubated for 2 min at room temperature, before placing it back on the magnet until the solution was clear. Then 20 μL of the supernatant (purified sample) were transferred to a new plate. To remove unprocessed adapters a second round of purification was performed. 20 μl of Ampure Beads (equilibrated to RT) were transferred to each well containing the purified sample from the first cleanup step. The plate was sealed, vortexed and incubated 5 min at room temperature and placed on a magnet until the solution was clear. The supernatant was removed from each well and 200 μl freshly prepared 80% ethanol was added to each well, incubated 30 sec and removed. The washing step was repeated for a total of two washes. Then the EtOH was removed and evaporated for 3 min at RT. The plate was removed from the magnet and each bead pellet was resuspended in 22 μL nuclease-free water. The plate was incubated for 2 min at room temperature, before placing it back on the magnet until the solution was clear. Then 20 μL of the purified sample were transferred to a new plate.

Library Amplification and Strand Selection

For the final amplification the Equinox Amplification Master Mix (2X) was thawed on ice and inverted several times.

Library Amplification Program

| Step | Temperature | Time | Cycles |
|---|---|---|---|
| Initial denaturation | 98°C | 45 sec | 1 |
| Denaturation | 98°C | 15 sec | |
| Annealing | Indexed primers: 55°C | 30 sec | 18 cycles |
| Extension | 72°C | 30 - 45 sec | |
| Final extension | 72°C | 60 sec | 1 |
| Hold | 12°C | Hold | |

For each reaction the Library Amplification Master Mix was prepared as follows:

| Component | Volume (μl) |
|---|---|
| Adapter-ligated library | 20 |
| IDT xGen™ UDI Primers Plate 8nt | 5 |
| Equinox Amplification Master Mix (2X) | 25 |

After the index adapter primers and the Equinox Amplification Master Mix was added to each well, the reaction was mixed by pipetting it up and down 10 x.

The plate was placed in a thermocycler and the Library Amplification program was initiated.

Once the program was completed, the protocol was immediately proceeded to Post-amplification Cleanup.

Post-amplification Clean-up

80% EtOH was freshly prepared (0.4ml for each reaction. The Ampure XP Beads were equilibrated to room temperature and mixed by vortexing. 50 μl Ampure XP Beads were added to each well. The plate was sealed and mixed on a vortexer followed by 5 min incubation at room temperature. After that the plate was placed on a magnet until the solution was clear. The supernatant was removed from each well and 200 μl freshly prepared 80% ethanol was added to each well, incubated 30 sec and removed. The washing step was repeated for a total of two washes. Then the EtOH was removed and evaporated for 3 min at RT. The plate was removed from the magnet and each bead pellet was resuspended in 22 μL nuclease-free water. The plate was incubated for 2 min at room temperature, before placing it back on the magnet until the solution was clear. Then 20 μL of the purified sample were transferred to a new plate.

Quality Control

To assess the library quality and quantity the library was checked with a DNA 1000 Screentape on the Tapestation (Agilent). The Tapestation profiles were inspected for the average library size to be around 320 - 420 bp without adapter dimer peaks (~200 bp). Furthermore, the libraries were quantified using the 1 x DNA High Sensitivity assay and the Qubit Flex (Life Technologies). The nanomolarity of each library was calculated based on the average size (Tapestation) and the concentration (Qubit) according to the following formula:

$$\frac{(\text{concentration in ng/μl})}{(660 \text{ g/mol} \times \text{average library size in bp})} \times 10^6 = \text{concentration in nM}$$

The libraries were pooled equimolarly, and the concentration of the final pool was measured using the Qubit 1 x DNA High Sensitivity assay and the Qubit (Life Technologies). The final pool was then sequenced using Illumina sequencing technology.

**Example 5: RNA input for Library Preparation**

Since the RNA yield, especially for filter cards from the real-life setting, can sometimes be low, we tested different total RNA input amounts for the library preparation. Test criteria were the successful generation of a library and that our general sequencing quality, i.e. total number of paired reads ≥20M, unique assigned reads ≥4M unique assigned reads, number of genes ≥8000.

We tested low (25ng), medium (50ng) and high (100ng) RNA inputs from dried blood spots.

The samples were prepared according to the protocols (Example 1, Example 4, Example 6) and the correlation of normalized read counts for the different input amounts and samples was calculated.

The correlation between the different RNA input amounts (50ng vs. 100ng, 25ng vs. 50ng, 25ng vs. 100ng) for the different filter card samples that was analyzed. The lowest correlation was 0.998 which proves that there are no relevant differences between low and high inputs and 25ng is sufficient as input for our library preparation protocol.

**Example 6: Sequencing and Data Processing**

The final pool was sequenced on a NextSeq 500 or NovaSeq 6000 device (both Illumina). For the NextSeq 500 the High Outut Kit v2.5 was used. Up to 30 samples were pooled for a sequencing run on the NextSeq. For the sequencing on the NovaSeq 6000 the S1 Reagent Kit v1.5. On the S1 flow cell up to 96 samples in total were pooled for one run. For the sequencing 2 different run cycle protocols were used. The 75 paired-end protocol refers to a sequencing length of 75 bases, where every fragment is sequenced from both sides resulting in two reads per fragment. The alternative protocol tested was the 150 paired-end protocol that generates longer reads. The average total number of reads over all runs was 20 M paired reads.

After sequencing the data was routinely processed according to the following pipeline to generate the gene counts which are the basis for all subsequent bioinformatic RNA sequencing analyses.

Generating gene counts is based on known (publicly available) pipelines for Illumina NGS analysis. Briefly, the photos generated from the sequencer (bcl files) were converted to text sequence (fastq file) using bcl2fastq tool. STAR2.7 was used to align the reads to the human reference genome (hg19), then PCR duplicates were removed (e.g. SAMtools and PICARD tools). Read counts were generated via counting tools (e.g. HTSseq and featureCount), normalized counts were generated by normalization tools (e.g. Stringtie2, STAR2.7, DESeq2). Bioinformatics QC was generated using several RNA-seq QC tools (FastQC, MultiQC, RNA-QC). Samples below QC cut-off (total number of reads >20M, number of unique assigned reads > 4M) were removed from further analysis. Samples were then further normalized, if necessary, based on the experimental design (by Gender, Age etc.)

Sample correlation was above 0.999 between the two methods, yielding to the conclusion that the method is repeatable and platform independent.

After the alignment and gene counts, differential gene expression (DEG) is performed by tools like DESeq2 or EdgeR. A list of genes is obtained that have statistically different gene expression in patients than healthy, or in treated than untreated or any other two conditions. This list of genes is investigated for enriched pathways in tools such as Toppgene, PantherDB or David. Alternatively, gene set enrichment analysis (GSEA) is a popular tool to search for enriched pathways between different condition when compared to common genesets (Reactome, KEGG, Wikipathways...). In addition to DEG and pathway enrichment, RNA-seq data can be analyzed for changes with time (different time points- longitudinal study) or with different doses (response to therapy). They can be called time course analysis among other labels. Many tools can be used to do time course analysis including TCseq, DESeq2 and DEGreport.

**Example 7: Gene correlation between RNA-seq from Blood vs from DBS**

To investigate the comparability between a regular blood sample (EDTA) and the corresponding DBS filter card prepared from the same blood tube the correlation between the gene counts was calculated.

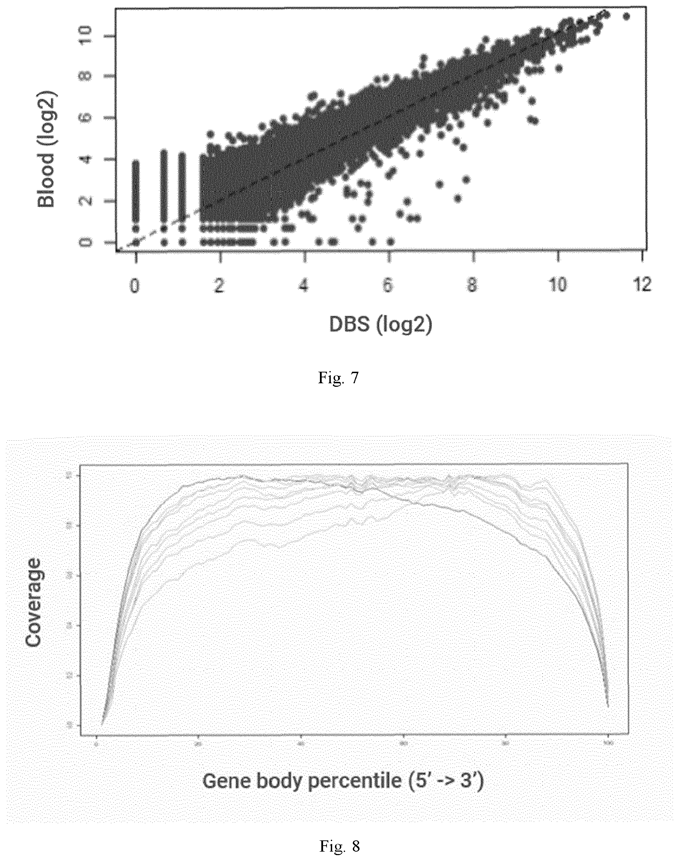

Figure 7 shows the results obtained from the first protocol version that was only based on the extraction protocol according to Example 1, a Poly(A) selection (Illumina stranded mRNA Kit) and a subsequent globin gene depletion (QIAseq Fast Select globin Kit). The result presented in Figure 7 is showing a high correlation between blood RNA and DBS RNA of 0,91.

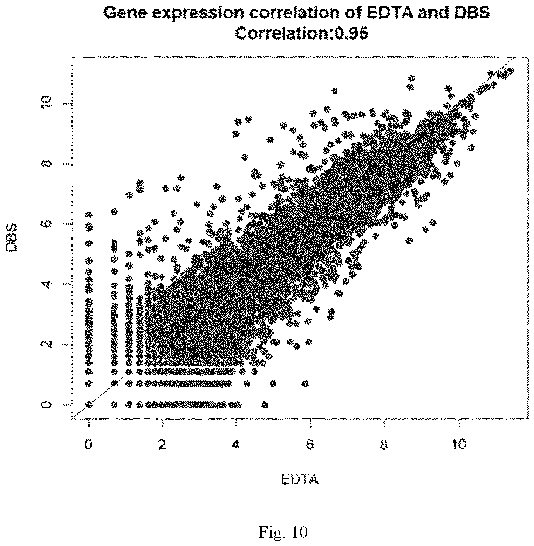

Due to deviations in the blood volume dropped onto the filter cards by the physicians the RNA yield was below 50 ng for a high number of samples. The final library preparation protocol (see Example 4) only required a minimum of 25 ng RNA. A Poly(A) selection and subsequent rRNA/globin depletion was still showing high correlations between Blood and DBS samples even though less total RNA starting material was used (see Figure 10). The result presented in Figure 10 is showing a high correlation between blood RNA and DBS RNA of 0,95 (slightly improved in comparison to the first protocol version).

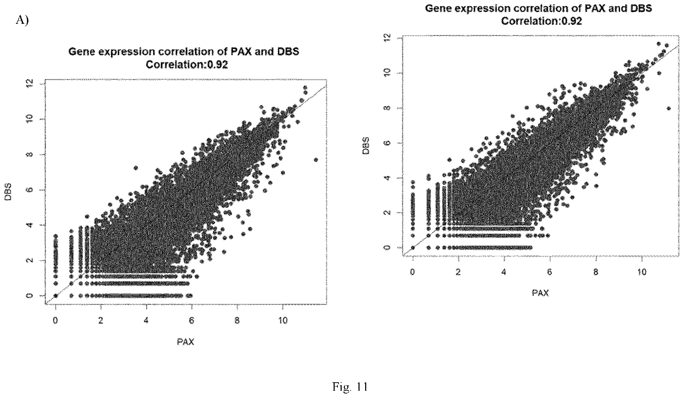

Today's gold standard for RNA analysis from blood is the PAXgene Blood tube that contains a reagent that is preventing RNA degradation. Therefore, it was also tested how high the gene expression correlation was between DBS RNA obtained with the herein described protocol (Example 1 and Example 4) and RNA obtained from PAXgene Blood RNA tubes using the PAXgene Blood RNA Kit. The result presented in Figure 11 is showing a high correlation between PAXgene blood RNA and DBS RNA of 0.87 and 0.88, respectively.

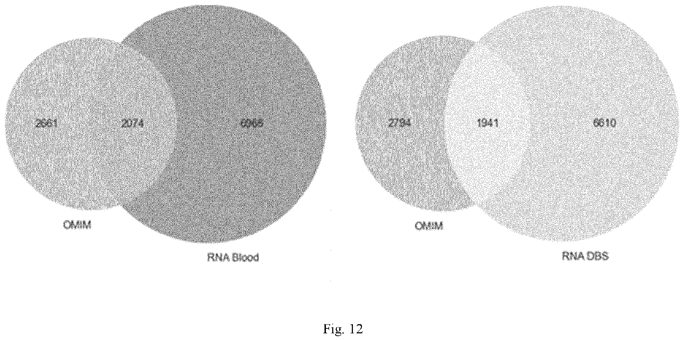

The data quality of RNA sequencing from dried blood spots is comparable to RNA extracted from commonly used blood collection products, e,g, PAXgene RNA blood or EDTA blood. RNA-seq from blood covers around 43% of the OMIM genes (genes with a known phenotype-causing mutation) with good coverage (Reads Per Kilobase Million- RPKM > 4). Figure 12 shows that mRNA sequencing from dried blood spots covered almost the same number of OMIM genes (~41%). The result presented in Figure 12 is showing a high correlation for gene coverage between blood RNA and DBS RNA.

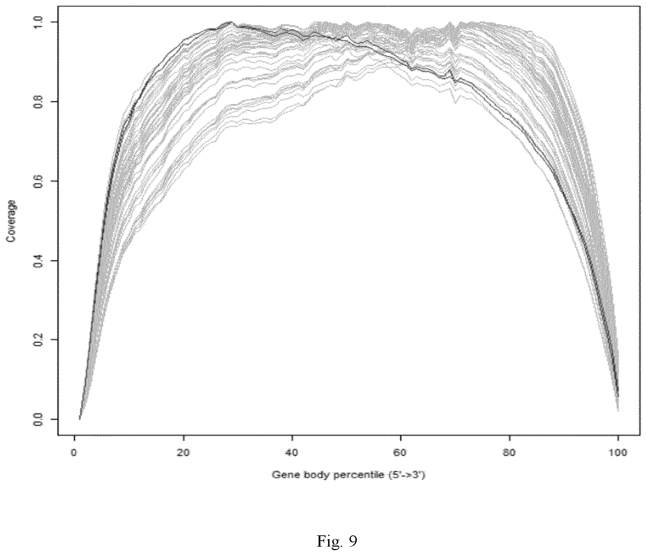

As shown in Example 3 the extraction of RNA filter cards resulted in a broad range of RIN values. For library preparation only samples with a RIN of 2.5 or higher were selected. Samples with such low RIN values are suspected to be highly degraded and would potentially decrease the coverage of 5' end of transcripts (PMID: 24632678). The gene body coverage was less affected than expected from literature as shown in Figure 8 (results obtained from the first protocol version that was only based on the extraction protocol according to Example 1, a Poly(A) selection (Illumina stranded mRNA Kit and a subsequent globin gene depletion (QIAseq Fast Select globin Kit)) and Figure 9 (improved protocol according to Example 1 and Example 4).

These data show that RNA obtained from blood samples (e.g. EDTA blood samples) or DBS (using the protocol according to example 1 and 4) show comparable results in data quality of RNA sequencing that proves that RNA preparation from DBS (using the protocol according to example 1 and 4) can be used in diagnostics.

**Example 8: Use cases**

**Example 8.1: Variant effect on mRNA structure**

Variants may cause changes in the structure of mRNA that affect its splicing, stability, folding and translation efficacy. Many patients remain undiagnosed with variants of unknown significance (VUS) or no relevant variants detected after exome or genome sequencing. The effects of VUS on transcript splicing can be variable and depend on the specific location and nature of the variant within the gene. While there are power tools to predict the effects of VUS, studying the RNA expressed with the variants is the confirmation. RNA-seq can be a powerful tool for the diagnosis of genetic diseases. It is particularly useful to assess the impact of variants at the mRNA level.

Variant effects on mRNA structure ranges from alternative splicing to decay of the transcript, changes in efficacy of translation to alternation in polyadenylation.

RNA splicing is a critical process that generates different mRNA isoforms from a single gene, allowing to produce multiple protein products with diverse functions. RNA-seq data can be used to identify and quantify alternative splicing by analyzing splice junction reads and by examining exon usage. Splice junction reads are generated when RNA-seq reads overlap two adjacent exons, indicating the presence of a spliced transcript isoform. By counting the number of reads that span each splice junction, scientists can quantify the expression levels of different transcript isoforms and identify alternatively spliced exons.

Some variants can alter the stability of the mRNA by changing the secondary structure or exposing regions that are more susceptible to degradation by ribonucleases. This can result in differences in the levels of mRNA expression or affect the half-life of the mRNA. Decaying RNA fragments have lower transcript count, thus influencing protein function.

103 patients for whom splicing variants had been reported were selected for RNA sequencing, considering the gene expression in blood and sample availability. The filter cards were kept at - 80°C for different periods of time (up to 3 month) and RNA was extracted (see Example 1). For all samples with a RIN>2.5 RNA libraries were generated (see Example 4) and sequencing on Illumina NextSeq500 or NovaSeq 6000 device was performed, followed by a data processing including alignment to the reference genome hg19 (see Example 6).

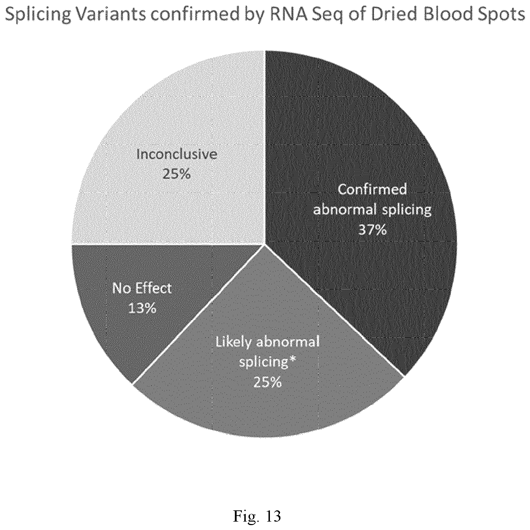

Splicing in genes of interest was visually inspected in the resulting bam files using IGV (Integrative genomic viewer) with at least two unaffected individuals as controls. Splicing in genes of interest was inspected using the Integrated genomics viewer (IGV) with at least two unaffected individuals as controls. Abnormal splicing was clearly detected in 40 samples (37%). Abnormal splicing was likely in 27 additional variants, but detected in a low number of reads, warranting an independent confirmation (re-sequencing). No evidence of abnormal splicing was detected (presence of two alleles was confirmed) in 14 samples. Inconclusive results were noticed in 27 cases (25%) given that number of reads in the gene of interest was not enough to confirm or exclude an effect on splicing (see Figure 13).

The confirmed abnormal splicing effects comprised: i) intron inclusion, ii) exon(s) skipping and iii) partial exon deletion.

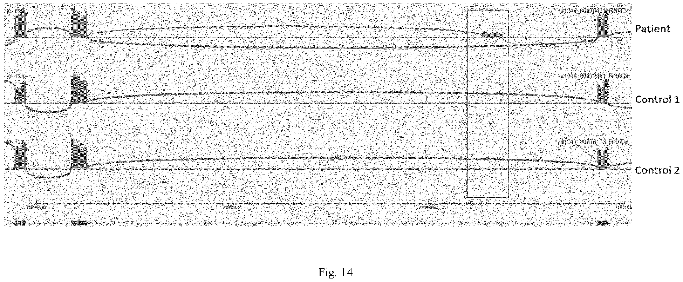

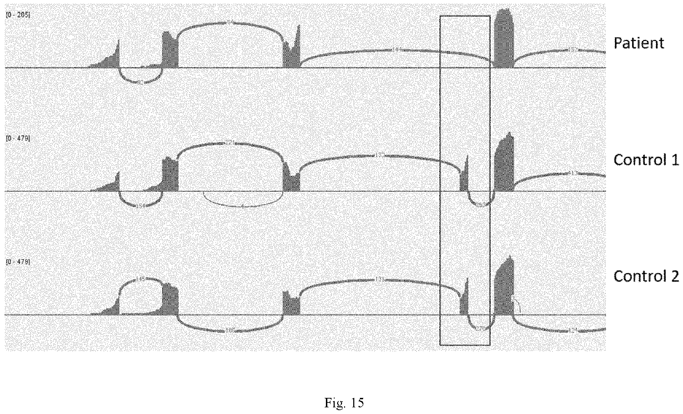

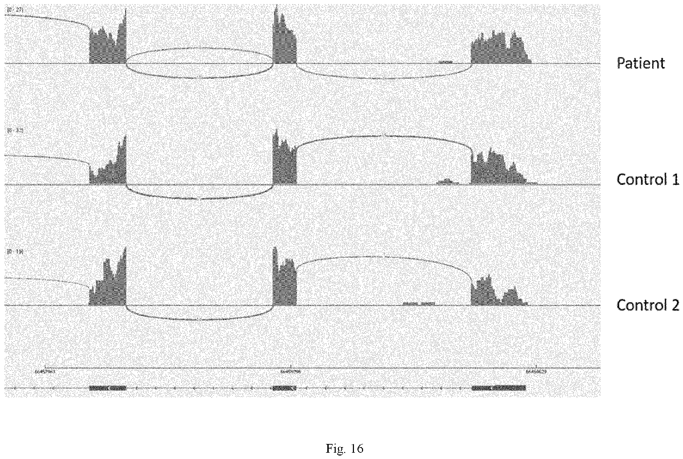

In Figure 14 results are presented for the observed splicing effects. Figure 13 shows the Sashimi plot of a heterozygous, deep intronic variant in the DYSF gene (c.5785-824C>T) resulting in an additional splice site causing the inclusion of the intron (formation of a cryptic exon). In Figure 14 a homozygous splice site variant in the DNAJB2 gene is presented *(c.176-2A>G)*. The Sashimi plot shows that Exon 04 is skipped in all reads leading to a shorter transcript. In Figure 15 and Figure 16 a heterozygous variant in the gene SBDS (c.258+2T>C) is shown. The splice site variant causes partial loss of the canonical splice donor site.

Overall, the results proof that splicing variants can be successfully detected in RNA sequencing data from dried blood spots helping to increase the diagnostic yield without the burden of additional sample collection from the patient.

**Example 8.2: Pathway enrichment**

RNA-seq can measure the sum of variant mechanisms controlling gene expression levels: Epigenetic modifications, RNA process machinery, RNA stability, Regulatory elements and cellular signaling.

The change in one gene expression is usually accompanied by changes in related pathways. RNA-seq can be used to measure pathway enrichment by comparing the expression of genes in a particular pathway to the expression of genes in the background of all expressed genes in DBS. To perform pathway enrichment analysis, Gene expression quantification is performed, then statistical gene set enrichment analysis using existing pathway databases.

By using RNA-seq to measure pathway enrichment, insights can be gained into the biological processes that are altered in a particular sample, such as the effects of a drug treatment, disease state, or environmental exposure. This can help to identify potential therapeutic targets and biomarkers, and aid in the development of personalized medicine.

Pathway enrichment analysis requires a high number of samples to uncover underlying causes of rare diseases.

The most practical way to collect high numbers of RNA samples is using DBS.

The gene set enrichment analysis (GSEA) algorithm calculates an enrichment score reflecting the degree of overrepresentation at the top or bottom of the ranked list of the genes included in a gene set in a ranked list of all genes present in the RNA-seq dataset. Gene expression data and the metadata are transformed in get and cls format. Gene expression is tested against gene sets such as Reactome, KEGG and WikiPathways. Number of permutations is minimum 1000, and no collapse option is selected. Other parameters depend on the experiment settings. The results of enriched pathways are analyzed for reliable FDR values.

To proof the suitability of filter cards for pathway analyses the differential gene expression between Parkinson's disease patients (n= 157) and controls (n=176) was explored. The Parkinson cohort contained of the following subgroups: i) with a GBA variant (n=56), ii) without GBA variant (n=6), iii) idiopathic (n=95),

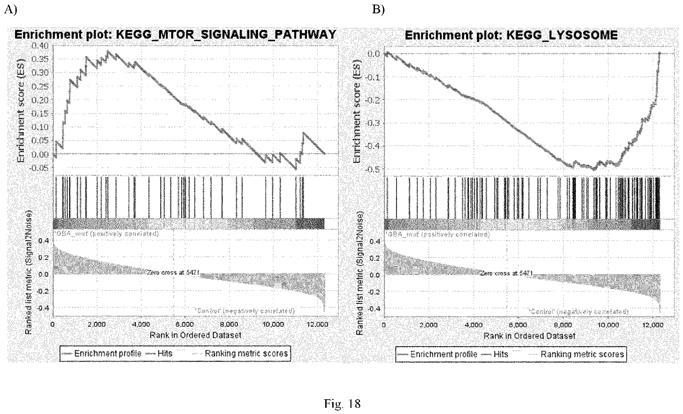

RNA extraction (see Example 1), library preparation (see Example 4) and sequencing (see Example 6) were performed for a total of 365 samples. Gene set enrichment analysis was performed for the group of Parkinson patients carrying a GBA variant and control samples. Figure 18 is showing 2 examples of the GSEA results. The upregulation of MTOR signaling pathway and downregulation of the lysosome pathway has already been described in the literature (PMID: 27263112, PMID: 30744070).

The presented data prove that the herein described protocols produce a data quality that is sufficient to detect meaningful pathway changes when comparing patients vs controls. The here presented pathways were documented already in the literature and especially mTOR signaling is known as a therapeutic target in Parkinson's disease.

**Example 8.3: Allele penetrance**

Allele penetrance refers to the degree to which a genetic variant (allele) is expressed in an individual's phenotype. There are some cases in which it may be possible to detect allele penetrance from RNA-seq data, particularly if the genetic variant in question is located within a coding region of a gene and produces a transcript that can be distinguished from other transcripts. In these cases, it may be possible to use RNA-seq data to compare the expression of the two alleles and to determine the degree to which each allele contributes to the overall phenotype.

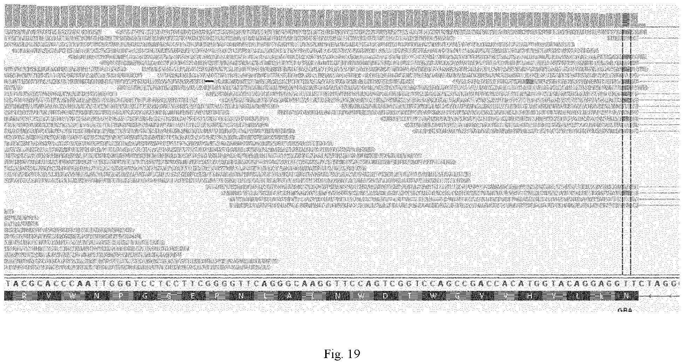

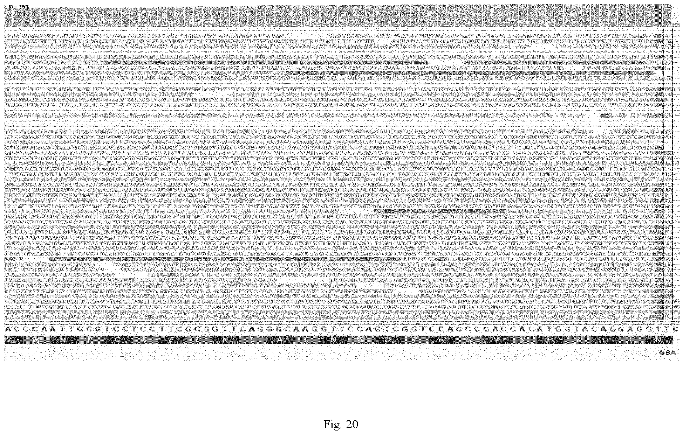

Samples for examining the allelic balance were prepared according to Example 1, a Poly(A) selection (Illumina stranded mRNA Kit), subsequent globin gene depletion (QIAseq Fast Select globin Kit) and Example 6 (Figure 19) or to Example 1, Example 4 and Example 6 (Figure 20). The bam files aligned to hg19 were visually inspected via IGV.

Figure 19 and Figure 20 present a heterozygous variant in the GBA gene. The allele frequency observed in IGV shows a balanced frequency for the patient shown in Figure 19. Figure 20 is an example for allelic imbalance. The heterozygous variant has a frequency of 70% on the mRNA level.

The presented data prove that the herein described protocols produce a data quality that is sufficient to determine the degree to which a genetic variant (allele) is expressed in an individual's phenotype.

**Example 8.4: Longitudinal studies and treatment follow-up**

In addition to diagnosis, RNA-seq can also be used to gain insights into the underlying molecular mechanisms of rare diseases, which can inform the development of new therapies and treatments. By identifying key genes and pathways that are dysregulated in a rare disease, researchers can develop targeted therapies that aim to correct these defects and improve patient outcomes.

RNA-seq can be used to measure gene expression changes over time, which can be particularly useful for monitoring the effects of treatments on gene expression. For example, in a study of treated patients with a particular disease, DBS samples can be collected from patients before treatment, and then at regular intervals after treatment. By analyzing the RNA-seq data from these samples, changes can identify in gene expression levels that are associated with the treatment.

To prove that treatment effects on gene expression can be monitored using RNA from dried blood spots we selected 6 consented individuals that were about to get their 2$^{nd}$ covid19 vaccination (Pfizer/Biontech or Moderna). Blood collection, filter card preparation and RNA extraction were conducted as described in Example 1. The generation of mRNA libraries was performed according to Example 4. Sequencing and data processing according to Example 6.

In addition to the generation of the read counts a time course analysis was performed to study the response to vaccination. Clustering genes in time course analysis can be performed via a variety of tools including TCSeq. Normalized gene expression can be obtained from DESeq2 normalized counts function.

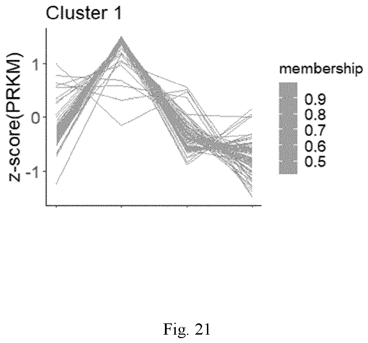

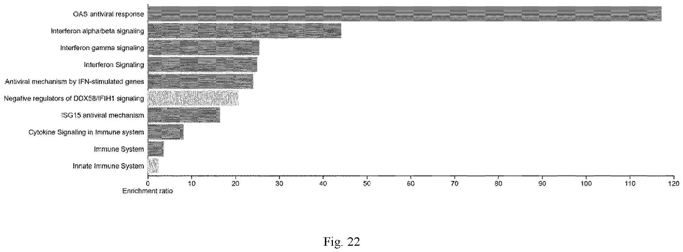

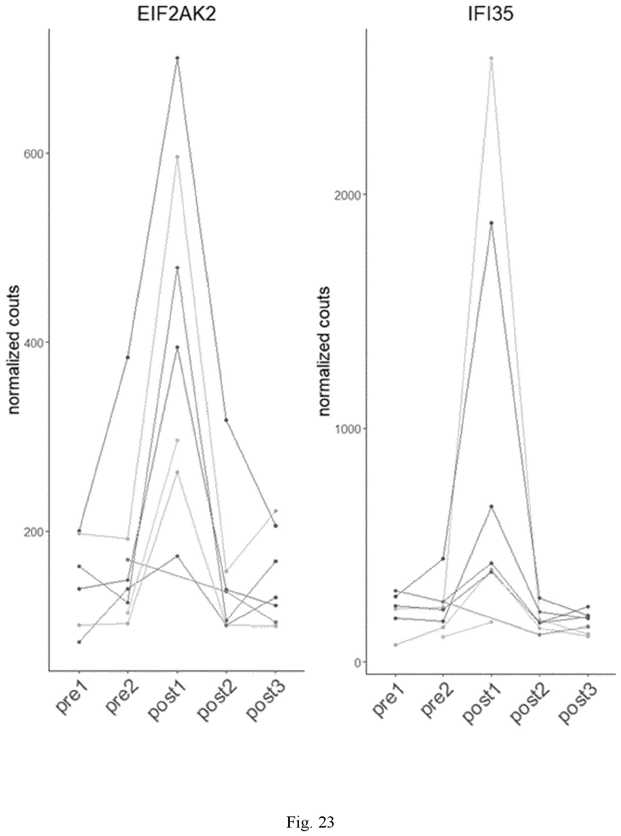

The time course analysis revealed several clusters of genes that behave similarly, e.g., increased at early stage then decreased later (Figure 21). A list of genes in each cluster was tested for pathway enrichment using ToppGene (Figure 22). Some genes increased immediately after the vaccination (post 1) then return to normal expression. There were also genes identified that increased after one week from vaccination (post3). The genes that increased immediately after vaccination are enriched in early immune response and interferon pathway. Examples of that are shown in Figure 23.

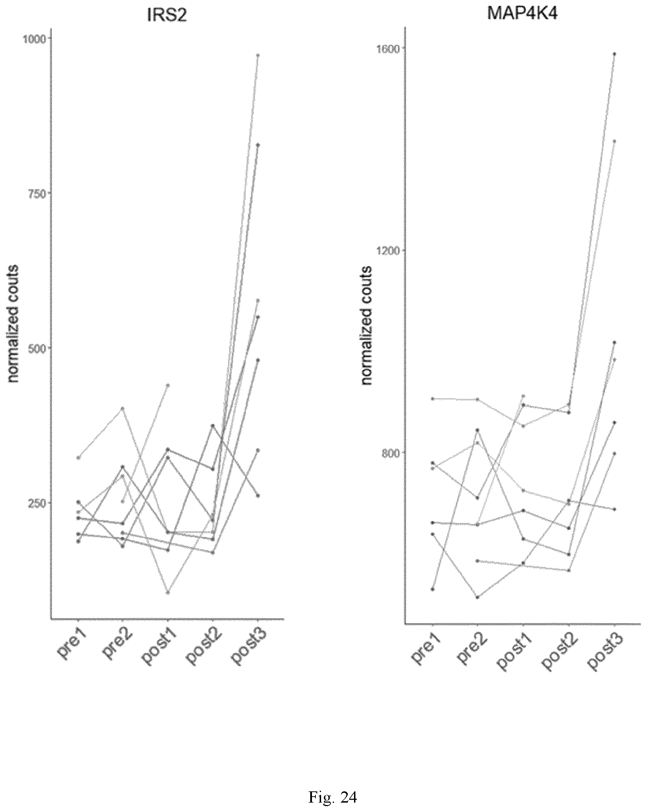

Another group of genes that increased only after a week of vaccination are enriched for late immune response pathways (Figure 24). The late response to the 2nd vaccination was the upregulation of genes from the insulin pathway (Figure 22). Other studies showed similar observations (PMID: 36793809, PMID: 33927933).

The presented data prove that the herein described protocols produce a data quality that is sufficient to measure gene expression changes over time, which can be particularly useful for monitoring the effects of treatments on gene expression.

The features of the present invention disclosed in the specification, the claims, the sequence listing and/or the drawings may both separately and in any combination thereof be material for realizing the invention in various forms thereof.

**Claims**

1. A method for preparing an RNA preparation, wherein the method comprises

    - extracting an RNA from a blood optionally comprising blood cells, wherein the blood is contained in a first sample, and/or extracting an RNA from blood cells contained in a first sample, wherein the RNA extracted from the blood optionally comprising blood cells and/or the RNA extracted from the blood cells is provided as a second sample, and

    - depleting an RNA from the second sample, wherein the RNA obtained after depleting the RNA from the second sample, is provided as the RNA preparation,

    wherein

    the first sample is a dried blood sample comprising the blood optionally comprising blood cells and/or the first sample is a dried blood sample comprising the blood cells,
    extracting the RNA from the blood optionally comprising blood cells and/or extracting the RNA from the blood cells comprises lysing the blood cells optionally comprised by the blood and/or lysing the blood cells, digesting protein contained in the first sample and/or digesting protein released from the blood cells optionally comprised by the blood and/or from the blood cells, and protecting from degradation RNA released from the blood cells optionally comprised by the blood and/or from the blood cells,
    depletion of the RNA from the second sample comprises depletion of hemoglobin RNA, depletion of rRNA and capturing of RNA by poly(A) tail capturing,
    lysing the blood cells optionally comprised by the blood and/or lysing the blood cells comprises lysing by protein digestion in combination with an agitation method, and
    protecting RNA from degradation is effected by inhibiting RNAses or by inhibiting RNAses and DNAses.

2. The method of claim 1, wherein the first sample is a dried blood sample comprising, dried on a filter paper, the blood optionally comprising blood cells and/or the first sample is a dried blood sample comprising the blood cells dried on a filter paper.

3. The method of any one of claims 1 to 2, wherein the blood optionally comprising blood cells is blood of a venous blood sample or a capillary blood sample and/or the blood cells are blood cells of a venous blood sample or a capillary blood sample, wherein the capillary blood sample was taken from a finger of the subject, preferably by use of a finger-prick device.

4. The method of any one of claims 1 to 3, wherein the RNA preparation is **characterized by** an RNA integrity number (RIN) >2, preferably an RNA integrity number (RIN) >2.5, more preferably an RNA integrity number (RIN) >3.

5. The method of any one of claims 1 to 4, wherein for preparing an RNA library from the RNA preparation at least 10 ng RNA of the RNA preparation are used, preferably at least 20 ng RNA of the RNA preparation are used, and more preferably at least 25 ng RNA of the RNA preparation are used.

6. A method of sequencing an RNA molecule in a sample, wherein the method comprises determining the nucleotide sequence of the RNA molecule and the sample is an RNA preparation prepared by a method as defined in any one of claims 1 to 5.

7. A method for quantifying an RNA molecule in a sample, wherein the method comprises determining an amount of the RNA molecule in the sample by means of quantitative polymerase chain reaction or next-generating sequencing, and wherein the sample is an RNA preparation prepared by a method as defined in any one of claims 1 to 5.

8. A method for determining the course of a disease in a subject, wherein the method comprises determining an amount of an RNA molecule in a sample from the subject at various points in time, wherein the RNA molecule is a prognostic marker for the disease and the sample is an RNA preparation prepared by a method as defined in any one of claims 1 to 5.

9. A method for monitoring an effect of a therapy administered to a subject suffering from a disease, wherein the method comprise determining an amount of an RNA molecule in a sample from the subject after the therapy was administered to the subject and at several points in time thereafter, wherein the amount of the RNA molecule is indicative of the effect of the therapy and wherein the sample is an RNA preparation prepared by a method as defined in any one of claims 1 to 5.

10. A method for determining whether a subject suffering from a disease is likely to respond to a treatment, wherein the method comprises detecting an RNA molecule in a sample from the subject, wherein the RNA molecule is a predictive marker for the disease and the sample is an RNA preparation prepared by a method as defined in any one of claims 1 to 5.

11. A method for determining the presence or absence of a splice variant of a gene in a sample, wherein the splice variant comprises a splice junction,

   wherein the method comprises determining whether the splice junction is present and if the splice junction is present, the splice variant is present and if the splice junction is absent, the splice variant is absent, and
   wherein the sample is an RNA preparation prepared by a method as defined in any one of claims 1 to 5.

12. A method for determining a relative abundance of an individual RNA molecule in a sample compared to the abundance of a plurality of RNA molecules in a sample, wherein the method comprises

   - determining an amount of the individual RNA in a sample, wherein the sample is an RNA preparation prepared by a method as defined in any one of claims 1 to 5, and
   - determining the relative amount of the individual RNA in a sample to the amount of at least one RNA molecule of the plurality of RNA molecules in a sample.

13. A method for determining allele-specific expression of a gene in a plurality of samples, wherein a plurality of alleles of the gene exist, wherein the method comprises

   - determining the nucleotide sequence of an RNA transcript of the gene in each and any sample of the plurality of samples,
   - allocating each nucleotide sequence to an allele of the plurality of alleles of the gene, and
   - calculating based on such allocation the relative abundance of an allele relative a different allele of the gene and/or to the plurality of alleles of the gene, and

   wherein the sample is an RNA preparation prepared by a method as defined in any one of claims 1 to 5.

14. A method for determining the proportion of subjects of a group of subjects carrying an allele of a gene that also expresses an associated trait, wherein a plurality of alleles of the gene exist, wherein the method comprises

   - determining the nucleotide sequence of an RNA transcript of the gene in a sample from each and any subject of the group of subjects,
   - allocating each nucleotide sequence to an allele of the plurality of alleles of the gene, and
   - calculating based on such allocation the relative proportion of subjects of the group of subjects that expresses the associated trait, and

   wherein the sample is an RNA preparation prepared by a method as defined in any one of claims 1 to 5.

Fig. 1

Stability of RNA integrity from DBS at room temperature

Fig. 2

Fig. 3

Fig. 4

Fig. 5

Fig. 6

Fig. 7

Gene body percentile (5' -> 3')

Fig. 8

Fig. 9

Fig. 10

A)

Fig. 11

Fig. 12

Splicing Variants confirmed by RNA Seq of Dried Blood Spots

Inconclusive
25%

Confirmed
abnormal splicing
37%

No Effect
13%

Likely abnormal
splicing*
25%

Fig. 13

Fig. 14

Fig. 15

Fig. 16

Fig. 17

Fig. 18

Fig. 19

Fig. 20

Fig. 21

Fig. 22

Fig. 23

Fig. 24

Europäisches
Patentamt

European
Patent Office

Office européen
des brevets

# EUROPEAN SEARCH REPORT

Application Number

EP 23 18 6083

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | REUST MARY J. ET AL: "Dried Blood Spot RNA Transcriptomes Correlate with Transcriptomes Derived from Whole Blood RNA", THE AMERICAN SOCIETY OF TROPICAL MEDICINE AND HYGIENE, vol. 98, no. 5, 9 May 2018 (2018-05-09), pages 1541-1546, XP093116970, US ISSN: 0002-9637, DOI: 10.4269/ajtmh.17-0653 * the whole document * | 1-14 | INV. C12Q1/6806 C12N15/10 |
| Y | JINSUNG JANG ET AL: "Comparative evaluation for the globin gene depletion methods for mRNA sequencing using the whole blood-derived total RNAs", BMC GENOMICS, BIOMED CENTRAL LTD, LONDON, UK, vol. 21, no. 1, 11 December 2020 (2020-12-11), pages 1-9, XP021285517, DOI: 10.1186/S12864-020-07304-4 * the whole document * | 1-14 | |
| Y | US 5 654 179 A (LIN LILY [US]) 5 August 1997 (1997-08-05) * the whole document * | 1-14 | TECHNICAL FIELDS SEARCHED (IPC) C40B C12N C12Q |
| Y | WO 2014/033326 A1 (QIAGEN GMBH [DE]) 6 March 2014 (2014-03-06) * the whole document * | 1-14 | |
| Y | WO 2014/099121 A1 (GEN ELECTRIC [US]) 26 June 2014 (2014-06-26) * the whole document * | 1-14 | |

-/--

The present search report has been drawn up for all claims

| Place of search | Date of completion of the search | Examiner |
|---|---|---|
| Munich | 16 January 2024 | Bradbrook, Derek |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

| | Europäisches Patentamt European Patent Office Office européen des brevets | **EUROPEAN SEARCH REPORT** | Application Number EP 23 18 6083 |

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | ALVAREZ M ET AL: "An mRNA and DNA co-isolation method for forensic casework samples", ANALYTICAL BIOCHEMISTRY, ACADEMIC PRESS, AMSTERDAM, NL, vol. 335, no. 2, 15 December 2004 (2004-12-15), pages 289-298, XP004642087, ISSN: 0003-2697, DOI: 10.1016/J.AB.2004.09.002 * the whole document * | 1-14 | |
| A | Anonymous: "All insights start with the sample. Your comprehensive guide for isolating top-quality RNA", , 1 September 2018 (2018-09-01), XP093117158, Retrieved from the Internet: URL:https://www.qiagen.com/us/resources/download.aspx?id=19988eb0-6df5-4d26-8435-33efe98197da&lang=en [retrieved on 2024-01-09] * the whole document * | 1-14 | |

TECHNICAL FIELDS SEARCHED (IPC)

The present search report has been drawn up for all claims

| Place of search | Date of completion of the search | Examiner |
|---|---|---|
| Munich | 16 January 2024 | Bradbrook, Derek |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

1

## ANNEX TO THE EUROPEAN SEARCH REPORT
## ON EUROPEAN PATENT APPLICATION NO.

EP 23 18 6083

This annex lists the patent family members relating to the patent documents cited in the above-mentioned European search report.
The members are as contained in the European Patent Office EDP file on
The European Patent Office is in no way liable for these particulars which are merely given for the purpose of information.

16-01-2024

| Patent document cited in search report | | Publication date | Patent family member(s) | | Publication date |
|---|---|---|---|---|---|
| US 5654179 | A | 05-08-1997 | NONE | | |
| WO 2014033326 | A1 | 06-03-2014 | AU | 2013310861 A1 | 12-02-2015 |
| | | | CA | 2880136 A1 | 06-03-2014 |
| | | | CN | 104603267 A | 06-05-2015 |
| | | | EP | 2893015 A1 | 15-07-2015 |
| | | | JP | 6440616 B2 | 19-12-2018 |
| | | | JP | 2015526100 A | 10-09-2015 |
| | | | SG | 10201701747Y A | 27-04-2017 |
| | | | SG | 11201500655V A | 28-05-2015 |
| | | | US | 2015232831 A1 | 20-08-2015 |
| | | | WO | 2014033326 A1 | 06-03-2014 |
| WO 2014099121 | A1 | 26-06-2014 | EP | 2935583 A1 | 28-10-2015 |
| | | | ES | 2692726 T3 | 04-12-2018 |
| | | | JP | 6544860 B2 | 17-07-2019 |
| | | | JP | 2016505262 A | 25-02-2016 |
| | | | WO | 2014099121 A1 | 26-06-2014 |

EPO FORM P0459

For more details about this annex : see Official Journal of the European Patent Office, No. 12/82